# EUROPÄISCHE PATENTSCHRIFT

(11) **EP 1 418 879 B1**
(45) Veröffentlichungstag und Bekanntmachung des Hinweises auf die Patenterteilung: **12.04.2006**
(21) Anmeldenummer: 02724298.1
(22) Anmeldetag: 24.04.2002
(51) Int. Cl.: A61K 8/41, A61K 8/40, A61K 8/49, A61Q 5/10, C07C 215/76, C07D 213/38, C07D 239/26

(54) **3-AMINOPHENOL-DERIVATE ENTHALTENDE OXIDATIONSHAARFÄRBEMITTEL SOWIE NEUE 3-AMINOPHENOL-DERIVATE**
OXIDATION HAIR DYES CONTAINING 3-AMINOPHENOL DERIVATIVES, AND NOVEL 3-AMINOPHENOL DERIVATIVES
COLORANTS D'OXYDATION CAPILLAIRES, RENFERMANT DES DERIVES DE 3-AMINOPHENOL, ET NOUVEAUX DERIVES DE 3-AMINOPHENOL

(30) Priorität: 25.08.2001 DE 10141722
(43) Veröffentlichungstag der Anmeldung: 19.05.2004
(73) Patentinhaber: Wella Aktiengesellschaft, 64274 Darmstadt (DE)
(72) Erfinder: PASQUIER, Cécile, CH-1723 Marly (CH); WYSS, Patrick, CH-1212 Grand-Lancy (CH); BRAUN, Hans-Jürgen, CH-3182 Überstorf (CH)
(74) Vertreter: Bockhorst, Matthias
(86) Internationale Anmeldenummer: PCT/EP2002/004490
(87) Internationale Veröffentlichungsnummer: WO 2003/017955

(56) Entgegenhaltungen:
- DE-A- 2 518 393
- DE-C- 10 032 134
- US-A- 5 019 130
- JACOBSEN, P; HÖNIGSBERGER, F: "Ueber das Metaoxyazobenzol und die Constitution der Paraoxyazokörper" CHEMISCHE BERICHTE, Bd. 36, 1903, Seiten 4093-4123, XP002214930
- HUBERT-HABART, M; PÈNE, C; BASTIAN, G; ROYER, R: "Recherches sur les dérivés nitrés d'intérêt biologique. VI. - Sur la synthèse d'(hydroxy-2 nitro-4 phényl)-5 pyrimidines à partir de dérivés nitrés de benzofurannes substitués en 3 par un groupement électro-attractif" CHIMIE TH¹RAPEUTIQUE, Bd. 8, Nr. 3, 1973, Seiten 314-318, XP001099069

## Beschreibung

Die vorliegende Erfindung betrifft Mittel zur oxidativen Färbung von Keratinfasern, insbesondere menschlichen Haaren, auf der Basis einer Entwickler/Kupplersubstanz-Kombination, welche als Kupplersubstanz, in 6-Stellung substituierte 3-Aminophenol-Derivate enthalten, sowie neue in 6-Stellung substituierte 3-Aminophenol-Derivate.

Auf dem Gebiet der Färbung von Keratinfasern, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol, 1,4-Diaminobenzol und 4,5-Diamino-1-(2-hydroxyethyl)-pyrazol eingesetzt, während als Kupplersubstanzen beispielsweise Resorcin, 2-Methyl-resorcin, 1-Naphthol, 3-Aminophenol, m-Phenylendiamin, 2-Amino-4-(2'-hydroxyethyl)amino-anisol, 1,3-Diamino-4-(2'-hydroxyethoxy)benzol und 2,4-Diamino-5-fluor-toluol zu nennen sind.

An Oxidationsfarbstoffe, die zur Färbung menschlicher Haare verwendet werden, werden neben der Färbung in der gewünschten Intensität zahlreiche zusätzliche Anforderungen gestellt. So müssen die Farbstoffe in toxikologischer und dermatologischer Hinsicht unbedenklich sein und die erzielten Haarfärbungen eine gute Lichtechtheit, Dauerwellechtheit, Säureechtheit und Reibeechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, dass durch Kombination geeigneter Entwicklersubstanzen und Kupplersubstanzen eine breite Palette verschiedener Farbnuancen erzeugt werden kann.

Obwohl bereits eine Vielzahl von Kupplersubstanzen bekannt, ist es mit den derzeit bekannten Färbemitteln nicht möglich, die an ein Färbemittel gestellten Anforderungen in jeder Hinsicht zu erfüllen. Es besteht daher weiterhin ein Bedürfnis nach neuen Kupplersubstanzen, welche die vorgenannten Anforderungen in besonderem Masse erfüllen.

Es wurde nunmehr gefunden, dass bestimmte 3-Aminophenol-Derivate gemäß der allgemeinen Formel (I) die an Kupplersubstanzen gestellten Anforderungen in besonders hohem Masse erfüllen und mit bekannten Entwicklersubstanzen farbstarke, außerordentlich lichtechte und waschechte Farbnuancen ergeben.

Gegenstand der vorliegenden Erfindung ist daher ein Mittel zur Färbung von Keratinfasern, wie zum Beispiel Wolle, Pelzen, Federn oder Haaren und insbesondere menschlichen Haaren, auf der Basis einer Entwicklersubstanz-Kupplersubstanz-Kombination, welches dadurch gekennzeichnet ist, dass es mindestens ein 3-Aminophenol-Derivat der Formel (I) oder dessen physiologisch verträgliche, wasserlösliche Salze enthält, 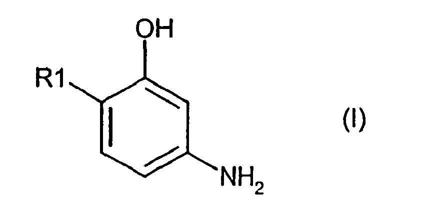 worin **R1** gleich einem Rest der Formel (II) oder (III) ist; 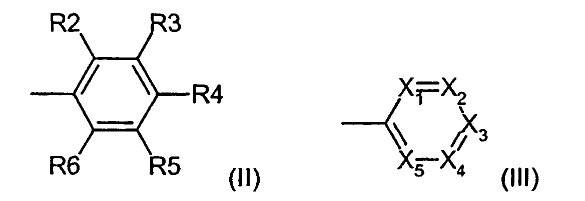 wobei **R2, R3, R4, R5** und **R6** unabhängig voneinander Wasserstoff, ein Halogenatom (F, Cl, Br, J), eine Cyanogruppe, eine Hydroxygruppe, eine C₁-C₄-Alkoxygruppe, eine Phenoxygruppe, eine C₁-C₄-Hydroxyalkoxygruppe, eine C₁-C₆-Alkylgruppe, eine Phenylgruppe, eine C₁-C₄-Alkylthioethergruppe, eine Mercaptogruppe, eine Nitrogruppe, eine Aminogruppe, eine C₁-C₄-Alkylaminogruppe, eine Hydroxy(C₂-C₄)alkylaminogruppe, eine Di(C₁-C₄)alkylaminogruppe, eine Di(hydroxy(C₂-C₄)alkyl)-aminogruppe, eine (Dihydroxy(C₃-C₄)alkyl)-aminogruppe, eine (Hydroxy(C₂-C₄)alkyl)-C₁-C₄-alkylaminogruppe, eine Trifluormethangruppe, eine -C(O)H-Gruppe, eine -C(O)CH₃-Gruppe, eine -C(O)CF₃-Gruppe, eine -Si(CH₃)₃-Gruppe, eine (C₁-C₄)-Hydroxyalkylgruppe, eine (C₂-C₄)-Dihydroxyalkylgruppe, eine (C₁-C₄)-Aminoalkylgruppe, oder eine (C₁-C₄)-Cyanoalkylgruppe darstellen, oder zwei nebeneinanderliegende Reste R2 bis R6 eine -O-CH2-O-Brücke bilden;
**X**_{**1**}**, X**_{**2**}**, X**_{**3**}**, X**_{**4**} und **X**_{**5**} unabhängig voneinander gleich Stickstoff oder einer C-R7-Gruppe, C-R8-Gruppe, C-R9-Gruppe, C-R10-Gruppe oder C-R11-Gruppe sind, unter der Bedingung, dass mindestens einer und höchstens drei der Reste **X1** bis **X5** Stickstoff bedeuten; und
**R7, R8, R9, R10 und R11** unabhängig voneinander Wasserstoff, ein Halogenatom (F, Cl, Br, J), eine Cyanogruppe, eine C₁-C₆-Alkylgruppe, eine (C₁-C₄)-Alkylthioethergruppe, eine Mercaptogruppe, eine Nitrogruppe, eine Aminogruppe, eine (C₁-C₄)-Alkylaminogruppe, eine Di(C₁-C₄)alkylaminogruppe, eine Trifluormethangruppe, eine -C(O)H-Gruppe, eine -C(O)CH₃-Gruppe, eine -C(O)CF₃-Gruppe, eine -Si(CH₃)₃-Gruppe, eine -C(O)-NH₂ Gruppe, eine (C₁-C₄)-Hydroxyalkylgruppe oder eine (C₂-C₄)-Dihydroxyalkylgruppe darstellen.

Als Verbindungen der Formel (I) können beispielweise genannt werden: 4-Amino-[1,1'-biphenyl]-2-ol, 4-Amino-4'-methyl- [1,1'-biphenyl]-2-ol, 4-Amino-3'-methyl-[1,1'-biphenyl]-2-ol, 4-Amino-2'-methyl-[1,1'-biphenyl]-2-ol, 4-Amino-2',3'-dimethyl-[1,1'-biphenyl]-2-ol, 4-Amino-2',4'-dimethyl-[1,1'-biphenyl]-2-ol, 4-Amino-2',5'-dimethyl-[1,1'-biphenyl]-2-ol, 4-Amino-2',6'-dimethyl-[1,1'-biphenyl]-2-ol, 4-Amino-3',4'-dimethyl-[1,1'-biphenyl]-2-ol, 4-Amino-3',5'-dimethyl-[1,1'-biphenyl]-2-ol, 4-Amino-3',6'-dimethyl-[1,1'-biphenyl]-2-ol, 4-Amino-2',4',5'-trimethyl-[1,1'-biphenyl]-2-ol, 4-Amino-2',4',6'-trimethyl-[1,1'-biphenyl]-2-ol, 4-Amino-2',3',4'-trimethyl-[1,1'-biphenyl]-2-ol, 4-Amino-2',3',5'-trimethyl-[1,1'-biphenyl]-2-ol, 4-Amino-2',3',6'-trimethyl-[1,1'-biphenyl]-2-ol, 4-Amino-4'-chlor-[1,1'-biphenyl]-2-ol, 4-Amino-3'-chlor-[1,1'-biphenyl]-2-ol, 4-Amino-2'-chlor-[1,1'-biphenyl]-2-ol, 4-Amino-4'-fluor-[1,1'-biphenyl]-2-ol, 4-Amino-3'-fluor-[1,1'-biphenyl]-2-ol, 4-Amino-2'-fluor-[1,1'-biphenyl]-2-ol, 4-Amino-4'-brom-[1,1'-biphenyl]-2-ol, 4-Amino-3'-brom-[1,1'-biphenyl]-2-ol, 4-Amino-2'-brom-[1,1'-biphenyl]-2-ol, 4-Amino-3',5'-dichlor-[1,1'-biphenyl]-2-ol, 4-Amino-3',5'-difluor-[1,1'-biphenyl]-2-ol, 4-Amino-3'-brom-5'-methyl-1,1'-biphenyl-2-ol, 4-Amino-4'-(trifluormethyl)-[1,1'-biphenyl]-2-ol, 4-Amino-3'-(trifluormethyl)-[1,1'-biphenyl]-2-ol, 4-Amino-2'-(trifluormethyl)-[1,1'-biphenyl]-2-ol, 4-Amino-4'-nitro-[1,1'-biphenyl]-2-ol, 4-Amino-3'-nitro-[1,1'-biphenyl]-2-ol, 4-Amino-2'-nitro-[1,1'-biphenyl]-2-ol, 4-Amino-5'-methyl-3'-nitro[1,1'-biphenyl]-2-ol, 4-Amino-4'-methyl-3'-nitro[1,1'-biphenyl]-2-ol, 4-Amino-2'-methyl-3'-nitro-[1,1'-biphenyl]-2-ol, 4-Amino-2'-nitro-4'-(trifluormethyl)[1,1'-biphenyl]-2-ol, 4-Amino-3'-nitro-5'-(trifluormethyl)[1,1'-biphenyl]-2-ol, 4-Amino-3'-nitro-4'-(trifluormethyl)[1,1'-biphenyl]-2-ol, 4-Amino-3'-nitro-2'-(trifluormethyl)[1,1'-biphenyl]-2-ol, 4'-Amino-2'-hydroxy-[1,1'-biphenyl]-4-carbonitril, 4'-Amino-2'-hydroxy-[1,1'-biphenyl]-3- carbonitril, 4-Amino-4'-methoxy-[1,1'-biphenyl]-2-ol, 4-Amino-3'-methoxy-[1,1'-biphenyl]-2-ol, 4-Amino-2'-methoxy-[1,1'-biphenyl]-2-ol, 4-Amino-4'-ethoxy-[1,1'-biphenyl]-2-ol, 4-Amino-3'-ethoxy-[1,1'-biphenyl]-2-ol, 4-Amino-2'-ethoxy-[1,1'-biphenyl]-2-ol, 4-Amino-3',4'-dimethoxy-[1,1'-biphenyl]-2-ol, 4-Amino-3',5'-dimethoxy-[1,1'-biphenyl]-2-ol, 4-Amino-2',3'-dimethoxy-[1,1'-biphenyl]-2-ol, 4-Amino-2',4'-dimethoxy-[1,1'-biphenyl]-2-ol, 4-Amino-2',5'-dimethoxy-[1,1'-biphenyl]-2-ol, 5-Amino-2-(1,3-benzodioxol-5-yl)-phenol, 4-Amino-4'-methoxy-3'-methyl-[1,1'-biphenyl]-2-ol, 4-Amino-4'-methoxy-2'-nitro-[1,1'-biphenyl]-2-ol, 4-Amino-4'-methoxy-3'-nitro-[1,1'-biphenyl]-2-ol, 4-Amino-4'-phenoxy-[1,1'-biphenyl]-2-ol, 4-Amino-4'-methylthio-[1,1'-biphenyl]-2-ol, 4-Amino-3'-methylthio-[1,1'-biphenyl]-2-ol, 4-Amino-2'-methylthio-[1,1'-biphenyl]-2-ol, 4-Amino-[1,1'-biphenyl]-2,4'-diol, 4-Amino-[1,1'-biphenyl]-2,3'-diol, 4-Amino-[1,1'-biphenyl]-2,2'-diol, 2,2',3'-Trihydroxy-4-amino-[1,1'-biphenyl], 2,2',4'-Trihydroxy-4-amino-[1,1'-biphenyl], 2,2',5'-Trihydroxy-4-amino-[1,1'-biphenyl], 2,2',6'-Trihydroxy-4-amino-[1,1'-biphenyl], 2,3',4'-Trihydroxy-4-amino-[1,1'-biphenyl], 2,3',5'-Trihydroxy-4-amino-[1,1'-biphenyl], 4-Amino-2'-methyl-[1,1'-biphenyl]-2.4'-diol, 2',4-Diamino-[1,1'-biphenyl]-2-ol, 3',4-Diamino-[1,1'-biphenyl]-2-ol, 4,4'-Diamino-[1,1'-biphenyl]-2-ol, 4,4'-Diamino-[1,1'-biphenyl]-2,2'-diol, 3',4-Diamino-[1,1'-biphenyl]-2,2'-ol, 3',4-Diamino-[1,1'-biphenyl]-2,4'-ol, 3',4-Diamino-[1,1'-biphenyl]-2,5'-ol, 3',4-Diamino-[1,1'-biphenyl]-2,6'-ol, 2',3',4-Triamino-[1,1'-biphenyl]-2-ol, 2',4,4'-Triamino-[1,1'-biphenyl]-2-ol, 2',4,5'-Triamino-[1,1'-biphenyl]-2-ol, 2',4,6'-Triamino-[1,1'-biphenyl]-2-ol, 3',4,4'-Triamino-[1,1'-biphenyl]-2-ol, 3',4,5'-Triamino-[1,1'-biphenyl]-2-ol, 1-(4'-Amino-2'-hydroxy-1,1'-biphenyl-4-yl)ethanon, 4-Amino-1,1':3',1"-terphenyl-2-ol, 4-Amino-1,1':4',1"-terphenyl-2-ol, 4-amino-4'-(aminomethyl)-1,1'-biphenyl-2-ol, 4-amino-3'-(aminomethyl)-1,1'-biphenyl-2-ol, 4-amino-2'-(aminomethyl)-1,1'-biphenyl-2-ol, (4'-amino-2'-hydroxy-1,1'-biphenyl-4-yl)acetonitril, (4'-amino-2'-hydroxy-1,1'-biphenyl-3-yl)acetonitril, (4'-amino-2'-hydroxy-1,1'-biphenyl-2-yl)acetonitril, 5-Amino-2-(4-pyridinyl)-phenol, 5-Amino-2-(3-pyridinyl)-phenol, 5-Amino-2-(2-pyridinyl)-phenol, 5-Amino-2-(3-methyl-2-pyridinyl)-phenol, 5-Amino-2-(4-methyl-2-pyridinyl)-phenol, 5-Amino-2-(5-methyl-2-pyridinyl)-phenol, 5-Amino-2-(6-methyl-2-pyridinyl)-phenol, 5-Amino-2-(3-chlor-2-pyridinyl)-phenol, 5-Amino-2-(4-chlor-2-pyridinyl)-phenol, 5-Amino-2-(5-chlor-2-pyridinyl)-phenol, 5-Amino-2-(6-chlor-2-pyridinyl)-phenol, 5-Amino-2-(3-fluor-2-pyridinyl)-phenol, 5-Amino-2-(4-fluor-2-pyridinyl)-phenol, 5-Amino-2-(5-fluor-2-pyridinyl)-phenol, 5-Amino-2-(6-fluor-2-pyridinyl)-phenol, 5-Amino-2-(3-trifluormethyl-2-pyridinyl)-phenol, 5-Amino-2-(4-trifluormethyl-2-pyridinyl)-phenol, 5-Amino-2-(5-trifluormethyl-2-pyridinyl)-phenol, 5-Amino-2-(6-trifluor methyl-2-pyridinyl)-phenol, 5-Amino-2-(3-nitro-2-pyridinyl)-phenol, 5-Amino-2-(4-nitro-2-pyridinyl)-phenol, 5-Amino-2-(5-nitro-2-pyridinyl)-phenol, 5-Amino-2-(6-nitro-2-pyridinyl)-phenol, 5-Amino-2-(2-methyl-3-pyridinyl)-phenol, 5-Amino-2-(4-methyl-3-pyridinyl)-phenol, 5-Amino-2-(5-methyl-3-pyridinyl)-phenol, 5-Amino-2-(6-methyl-3-pyridinyl)-phenol, 5-Amino-2-(2-chlor-3-pyridinyl)-phenol, 5-Amino-2-(4-chlor-3-pyridinyl)-phenol, 5-Amino-2-(5-chlor-3-pyridinyl)-phenol, 5-Amino-2-(6-chlor-3-pyridinyl)-phenol, 5-Amino-2-(2-brom-3-pyridinyl)-phenol, 5-Amino-2-(4-brom-3-pyridinyl)-phenol, 5-Amino-2-(5-brom-3-pyridinyl)-phenol, 5-Amino-2-(6-brom-3-pyridinyl)-phenol, 5-Amino-2-(2-nitro-3-pyridinyl)-phenol, 5-Amino-2-(4-nitro-3-pyridinyl)-phenol, 5-Amino-2-(5-nitro-3-pyridinyl)-phenol, 5-Amino-2-(6-nitro-3-pyridinyl)-phenol, 5-Amino-2-(5-pyrimidinyl)-phenol und 5-Amino-2-(4-pyrimidinyl)-phenol sowie deren physiologisch verträglichen wasserlöslichen Salze.

Bevorzugt sind Verbindungen der Formel (I) in denen:
(i) **R1** gleich einem Rest der Formel (II) mit **R2** und **R6** gleich Wasserstoff ist oder (ii) **R1** gleich einem Rest der Formel (III) mit **X1** und **X5** gleich **C-R7** und **C-R11** ist, wobei **R7** und **R11** gleich Wasserstoff sind.

Besonders bevorzugt sind die folgenden Verbindungen der Formel (I): 4-Amino-3'-methyl-[1,1'-biphenyl]-2-ol, 4-Amino-4'-methyl-3'-nitro[1,1'-biphenyl]-2-ol, 4-Amino-[1,1'-biphenyl]-2,4'-diol, 5-Amino-2-(3-pyridinyl)-phenol und 5-Amino-2-(5-pyrimidinyl)-phenol sowie deren physiologisch verträglichen wasserlöslichen Salze.

Die Verbindungen der Formel (I) können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Die 3-Aminophenol-Derivate der Formel (I) sind in dem erfindungsgemäßen Färbemittel in einer Gesamtmenge von etwa 0,005 bis 20 Gewichtsprozent enthalten, wobei eine Menge von etwa 0,01 bis 5 Gewichtsprozent und insbesondere 0,1 bis 2,5 Gewichtsprozent bevorzugt ist.

Als Entwicklersubstanzen kommen vorzugsweise 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diaminobiphenyl, 1,4-Diamino-2-methoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)-amino]-anilin, 4-[Di(2-hydroxyethyl)amino]-anilin, 4-[Di(2-hydroxyethyl)-amino]-2-methyl-anilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(1-hydroxyethyl)-benzol, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluor phenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methyl-phenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1H-pyrazol, 4,5-Diamino-1-[(4-methylphenyl)methyl]-1H-pyrazol, 1-[(4-Chlorphenyl)methyl]-4,5-diamino-1H-pyrazol, 4,5-Diamino-1-methyl-1H-pyrazol, 2-Amino-phenol, 2-Amino-6-methyl-phenol, 2-Amino-5-methyl-phenol und 1,2,4-Trihydroxy-benzol in Betracht.

Weiterhin kann das erfindungsgemäße Färbemittel zusätzlich zu den Verbindungen der Formel (I) noch weitere bekannte Kupplersubstanzen, beispielsweise N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-d!aminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion, enthalten.

Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5 Gewichtsprozent und insbesondere 0,1 bis 2,5 Gewichtsprozent, beträgt.

Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6 Gewichtsprozent besonders bevorzugt ist. Die Entwicklersubstanzen und Kuppler substanzen werden im allgemeinen in etwa äquimolaren Mengen eingesetzt; es ist jedoch nicht nachteilig, wenn die Entwicklersubstanzen diesbezüglich in einem gewissen Überschuß oder Unterschuß vorhanden sind.

Weiterhin kann das erfindungsgemäße Färbemittel zusätzlich andere Farbkomponenten, wie zum Beispiel 6-Amino-2-methylphenol und 2-Amino-5-methylphenol, sowie ferner übliche synthetische oder natürliche direktziehende Farbstoffe, beispielsweise Pflanzenfarbstoffe oder synthetische direktziehende Farbstoffe aus der Gruppe der sauren oder basischen Farbstoffe, der Triphenylmethanfarbstoffe, der aromatischen Nitrofarbstoffe, der Azofarbstoffe und der Dispersionsfarbstoffe, enthalten. Die erfindungsgemäßen Färbemittel können diese Farbkomponenten in einer Menge von etwa 0,1 bis 4 Gewichtsprozent enthalten.

Selbstverständlich können die zusätzlichen Kupplersubstanzen sowie die Entwicklersubstanzen und die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.

Die Zubereitungsform des erfindungsgemäßen Färbemittels kann beispielsweise eine Lösung, insbesondere eine wässrige oder wässrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Ubliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester femer Verdicker wie hohere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 30 Gewichtsprozent und die Pflegestoffe in einer Konzentration von etwa 0,1 bis 5 Gewichtsprozent.

Je nach Zusammensetzung kann das erfindungsgemäße Färbemittel schwach sauer, neutral oder alkalisch reagieren. Insbesondere weist es einen pH-Wert von 6,5 bis 11,5 auf, wobei die basische Einstellung vorzugsweise mit Ammoniak erfolgt. Es können aber auch Aminosäuren und/oder organische Amine, zum Beispiel Monoethanolamin und Triethanolamin, sowie anorganische Basen, wie zum Beispiel Natriumhydroxid und Kaliumhydroxid Verwendung finden. Für eine pH-Einstellung im sauren Bereich kommen anorganische oder organische Säuren, zum Beispiel Phosphorsäure, Essigsäure, Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12prozentigen, vorzugsweise 6prozentigen, wässrigen Lösung, aber auch Luftsauerstoff in Betracht. Wird eine 6prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugeweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Das erfindungsgemäße Färbemittel mit einem Gehalt an 3-Aminophenol-Derivaten der Formel (I) als Kupplersubstanz ermöglicht Färbungen mit ausgezeichneter Farbechtheit, insbesondere was die Lichtechtheit, Waschechtheit und Reibeechtheit anbetrifft. Hinsichtlich der färberischen Eigenschaften bietet das erfindungsgemäße Färbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre besondere Farbintensität aus. Die sehr guten färberischen Eigenschaften des Färbemittels gemäß der vorliegenden Anmeldung zeigen sich weiterhin darin, dass dieses Mittel insbesondere auch eine Anfärbung von ergrauten, chemisch nicht vorgeschädigten Haaren problemlos und mit guter Deckkraft ermöglicht.

Die Herstellung der erfindungsgemäßen Aminophenol-Derivate der Formel (I) kann unter Verwendung von literaturbekannten Syntheseverfahren erfolgen, beispielsweise
a) durch eine Tetrakis(triphenylphospin)palladium (0) katalysierte Kupplung eines geeignet substituierten 3-Aminophenol-borsäurederivates der Formel (IV) 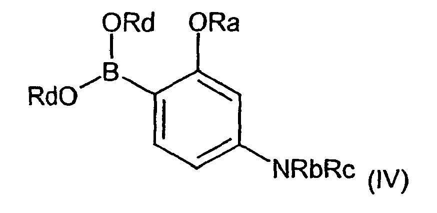 mit einer halogensubstituierten Verbindung der Formel (lla) beziehungsweise (llla) 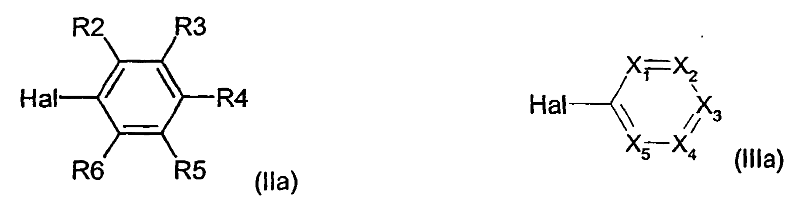 und anschließende Abspaltung der für die Kupplungsreaktion erforderlichen Schutzgruppen, oder
b) durch eine Tetrakis(triphenylphospin)palladium (0) katalysierte Kupplung eines halogensubstituierten 3-Aminophenol-Derivates der Formel (V) 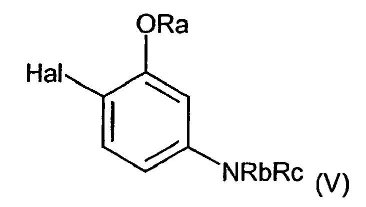 mit einem Borsäurederivat der Formel (IIb) beziehungsweise (IIIb) 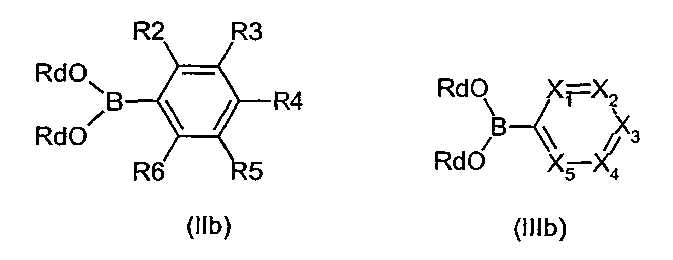 und anschließende Abspaltung der für die Kupplungsreaktion erforderlichen Schutzgruppen; wobei die in den Formeln (IIa), (IIb), (IIIa), (IIIb), (IV), und (V) verwendeten Restgruppen die folgende Bedeutung haben:
   **Ra** steht für eine Schutzgruppe, wie sie zum Beispiel in dem Kapitel "Protective Groups" in Organic Synthesis, Kapitel 3, Wiley Interscience, 1991 beschrieben wird;
   **Rb** und **Rc** stehen unabhängig voneinander für Wasserstoff oder eine Schutzgruppe, wie sie zum Beispiel in dem Kapitel "Protective Groups" in Organic Synthesis, Kapitel 7, Wiley Interscience, 1991 beschrieben wird;
   **Rd** ist gleich Wasserstoff oder die beiden Rd-Reste bilden gemeinsam mit der O-B-O-Gruppe einen unsubstituierten oder substituierten fünfgliedrigen oder sechsgliedrigen cycloaliphatischen Ring;
   **Hal** ist gleich F, Cl, Br oder J; und
   **R2, R3, R4, R5** und **R6** sowie **X**_{**1**}**, X**_{**2**}**, X**_{**3**}**, X**_{**4**} und **X**_{**5**} haben die in der Formel (II) beziehungsweise (III) angegebene Bedeutung.

Die 3-Aminophenol-Derivate der Formel (I) sind gut in Wasser löslich und ermöglichen Färbungen mit ausgezeichneter Farbintensität und Farbechtheit, insbesondere was die Lichtechtheit, Waschechtheit und Reibeechtheit anbetrifft. Sie weisen weiterhin eine ausgezeichnete Lagerstabilität, insbesondere als Bestandteil der vorgenannten Oxidationsfärbemittel, auf.

Ein weiterer Gegenstand der vorliegenden Erfindung sind neue 3-Aminophenol-Derivate der vorstehenden Formel (I), oder deren physiologisch verträglichen wasserlösliche Salze, mit der Bedingung, dass (i) **R1** kein 2-Pyridylrest ist und (ii) **R1** kein 2-Hydroxy-4-amino-phenylrest ist und (iii) **R4** nicht gleich einer Aminogruppe ist, wenn gilt **R2=R3=R5=R6=H.**

Die nachfolgenden Beispiele sollen den Gegenstand der Erfindung näher erläutern, ohne ihn darauf zu beschränken.

### Beispeile

### Beispiel 1 bis 45: Synthese von 3-Aminophenol-Derivaten der allgemeinen Formel (I)

### A. Synthese von 3-Ethoxymethoxy-phenylamin

Zu einer Lösung von 20,0 g (183,3 mmol) 3-Aminophenol in 450 ml getrocknetem Acetonitril gibt man bei 0 °C portionsweise 12 g (274,9 mmol) einer Natriumhydrid-Dispersion (55% in Öl). Das Gemisch wird anschließend 3 Stunden lang bei 0 °C gerührt. Dann gibt man tropfenweise eine Lösung von 25 g (210,8 mmol) Chlormethylethylether in 30 ml Acetonitril hinzu und rührt das Gemisch über Nacht bei Raum Temperatur. Anschließend wird das Reaktionsgemisch filtriert und der Filtrationsrückstand mit wenig Aceton gewaschen. Die vereinigten Filtrate werden eingeengt. Es werden 32,3 g 3-Ethoxymethoxy-phenylamin erhalten. Das erhaltene Rohprodukt wird ohne weitere Reinigung in der nächsten Stufe eingesetzt.
¹H-NMR (300 MHz, DMSO): δ= 6,89 (t, 1H, H5); 6,24 (s, 1H, H2); 6,22 (d, 1H); 6,16 (d, 1H); 5,14 (s; 2H, NH2); 5,11 (s, 2H, OCH2); 3,75 (q, 2H, CH₂); 1,13 (t, 3H, CH₃).

### B. Synthese von N-(3-Ethoxymethoxy-phenyl)-carbaminsäure-tert. butylester

30 g (180 mmol) 3-Ethoxymethoxy-phenylamin aus Stufe A und 44,4 g (203 mol) Di-tert-butyl-dicarbonat werden in einer Mischung von 140 ml 2N Natriumhydroxid und 200 ml Dichlormethan gelöst und 24 Stunden lang bei Raumtemperatur gerührt. Anschließend wird die organische Phase abgetrennt, mit einer gesättigten wässrigen Kochsalzlösung bis zu einem neutralem pH-Wert gewaschen, über MgSO₄ getrocknet, filtriert und eingeengt. Das erhaltene Rohprodukt wird an Kieselgel mit Hexan/Essigsäureethylester (8:1) gereinigt.

Es werden 18 g (42 % der Theorie, bezogen auf die eingesetzte Menge an 3-Aminophenol) N-(3-Ethoxymethoxy-phenyl)-carbaminsäure-tert.butylester als gelbes Öl erhalten.
¹H-NMR (300 MHz, DMSO): δ = 9,33 (s, 1H, NH); 7,20 (s, 1H, H2); 7,14 (t, J=8,0, 1H, H5); 7,05 (d, J=8,0, 1H, H3); 6,63 (d, J=8,0, 1H, H6); 5,17 (s, 2H, OCH2); 3,64 (q, J=7.1, 2H, CH2); 1,49 (s, 9H, t.butyl); 1,13 (t, J=7,1, 3H, CH3).

| CHN-Analyse: | | | | |
|---|---|---|---|---|
| (C₁₄H₂₁NO₄) | | % C | % H | % N |
| | berechnet: | 62,90 | 7,92 | 5,24 |
| | gefunden: | 62,60 | 8,04 | 4,97 |

### C. Synthese von N-(4-Brom-3-ethoxymethoxy-phenyl)-carbaminsäure tert.butylester

13,9 g (52 mmol)) N-(3-Ethoxymethoxy-phenyl)-carbaminsäure-tert.butylester aus Stufe B und 10,2 g (57,2 mmol) N-Bromsuccinimid werden unter Stickstoff in 400 ml 1,2-Dimethoxyethan gelöst und 3 Stunden lang bei Raumtemperatur gerührt. Anschließend wird die Reaktionsmischung auf 1000 ml Eis/Wasser gegossen und mit Essigsäureethylester extrahiert. Die organische Phase wird mit einer gesättigten wässrigen Kochsalz-Lösung gewaschen, über Magnesiumsulfat getrocknet und nach Filtration eingeengt. Das erhaltene Rohprodukt wird an Kieselgel mit Hexan/Essigsäureethylester (4:1) gereinigt.

Es werden 14,4 g (76 % der Theorie) N-(4-Brom-3-ethoxymethoxyphenyl)-carbaminsäure-tert.butylester als Öl erhalten.
¹H-NMR (300 MHz, DMSO): δ = 9,50 (s, 1H, NH); 7,45 (d, J=2,0, 1H, H2); 7,43 (d, J=8,6, 1H, H5); 7,04 (dd, J=2,0, J=8,6, 1H, H6); 5,24 (s, 2H, OCH2), 3,70 (q, J=7,1, 2H, CH2); 1,48 (s, 9H, t.butyl); 1,16 (t, J=7,1, 3H, CH3).

| CHN-Analyse: | | | | |
|---|---|---|---|---|
| (C₁₄H₂₀BrNO₄) | | % C | % H | % N |
| | berechnet: | 48,57 | 5,82 | 4,05 |
| | gefunden: | 47,82 | 5,87 | 3,77 |

### D. Synthese von [4-(5,5-Dimethyl-[1,3,2]dioxaborinan-2-yl)-3-ethoxymethoxy-phenyl]-carbaminsäure tert.butylester

10 g (28,8 mmol) N-(4-Brom-3-ethoxymethoxy-phenyl)-carbaminsäure tert.butylester aus Stufe C und 13 g (57,6 mmol) 5,5,5',5'-Tetramethyl-2,2'-Bi-[1,3,2-dioxaborinan] werden unter Argon in 260 ml Dioxan gelöst. Anschließend werden 2,11 g (2,88 mmol) [1,1'-Bis(diphenylphosphino)-ferrocen]dichlor-Palladium(II) und 8,48 g (86,4 mmol) Kaliumacetat zugegeben und die Reaktionsmischung 7 Stunden lang auf 80 °C erwärmt. Dann wird die Reaktionsmischung in 1,6 I Eis/Wasser gegossen und mit Essigsäureethylester extrahiert. Die organische Phase wird mit einer gesättigten wässrigen Kochsalz-Lösung gewaschen, über Magnesiumsulfat getrocknet und nach Filtration eingeengt. Das erhaltene Rohprodukt wird an Kieselgel mit Hexan/Essigsäureethylester (2:1) gereinigt.

Es werden 5,84 g (54 % der Theorie) [4-(5,5-Dimethyl-[1,3,2]dioxaborinan-2-yl)-3-ethoxymethoxy-phenyl]-carbaminsäure-tert.butylester erhalten.
¹H-NMR (300 MHz, DMSO): δ = 9,40 (s, 1H, NH); 7,41 (d, J=8,1, 1H, H5); 7,21 (s, 1H, H2); 7,07 (d, J=8,1, 1H, H6); 5,09 (s, 2H, OCH2), 3,69 (s, 4H, BOCH2); 3,66 (q, J=7,1, 2H, CH2); 1,48 (s, 9H, t.butyl); 1,18 (t, 3H, CH3); 0,95 (s, 6H, CH3).

### E. Synthese der 3-Aminophenole der Formel (I)

0,23 g (0,6 mmol) [4-(5,5-Dimethyl-[1,3,2]dioxaborinan-2-yl)-3-ethoxymethoxy-phenyl]-carbaminsäure-tert.butylester aus Stufe D und 0,78 mmol des entsprechenden Bromderivates werden unter Argon in 4 ml 1,2-Dimethoxyethan gelöst. Anschließend werden 0,07 g (0,06 mmol) Tetrakis-(triphenylphosphin)-palladium und 0,8 ml 2N Kalium-carbonat-Lösung zugegeben und die Reaktionsmischung auf 80 °C erwärmt. Nach Beendigung der Reaktion wird die Reaktionsmischung in 15 ml Essigsäureethylester gegossen, die organische Phase mit einer 1 N Natriumhydroxid-Lösung extrahiert und sodann mit Magnesiumsulfat getrocknet. Das Lösungsmittel wird am Rotationsverdampfer abdestilliert und der Rückstand an Kieselgel mit Hexan/Essigsäureethylester gereinigt.

Das so erhaltene Produkt wird in 2 ml Ethanol gelöst und mit 1 ml einer 2,9molaren ethanolischen Salzsäurelösung oder mit einer 4molaren Salzsäure in Dioxan versetzt. Die Reaktionsmischung wird anschließend auf 55 °C erwärmt. Nach Beendigung der Reaktion wird der Niederschlag abfiltriert, mit Ethanol (oder Dioxan) gewaschen und sodann getrocknet.
1. 4-Amino-[1,1'-biphenyl]-2-ol-hydrochlorid
Verwendetes Bromderivat: Brombenzol
Ausbeute: 0,041 g (31% der Theorie)
ESI-MS: 186 [M+H]⁺(100)

2. 4-Amino-4'-methyl-[1,1'-biphenyl]-2-ol-hydrochlorid
Verwendetes Bromderivat: 4-Bromtoluol
Ausbeute: 0,026 g (18% der Theorie)
ESI-MS: 200 [M+H]⁺ (100)

3. 4-Amino-3'-methyl-[1,1'-biphenyl]-2-ol-hydrochlorid
Verwendetes Bromderivat: 3-Bromtoluol
Ausbeute: 0,048 g (28% der Theorie)
ESI-MS: 200 [M+H]⁺ (100)
¹H-NMR (300 MHz, DMSO): δ = 10,15 (s, 1H, OH); 7,31 (s, 1H, H2'); 7,29 (m, 3H); 7,13 (d, J=8,1, 1H, H6'); 7,00 (d, J=1,7, 1H, H3); 6,83 (dd, J=1,7, J=8,1, 1H, H5); 3,60 (s, br, 3H, NH3⁺); 2,35 (s, 3H, CH3).

4. 4-Amino-2',3'-dimethyl-[1,1'-biphenyl]-2-ol-hydrochlorid
Verwendetes Bromderivat: 3-Brom-o-xylol
Ausbeute: 0,046 g (32% der Theorie)
ESI-MS: 214 [M+H]⁺(100)

5. 4-Amino-2',5'-dimethyl-[1,1'-biphenyl]-2-ol-hydrochlorid
Verwendetes Bromderivat: 2-Brom-p-xylol
Ausbeute: 0,046 g (32% der Theorie)
ESI-MS: 214 [M+H]⁺ (100)

6. 4-Amino-2',4'-dimethyl-[1,1'-biphenyl]-2-ol-hydrochlorid
Verwendetes Bromderivat: 6-Brom-m-xylol
Ausbeute: 0,033 g (17% der Theorie)
ESI-MS: 214 [M+H]⁺ (100)

7. 4-Amino-3',4'-dimethyl-[1,1'-biphenyl]-2-ol-hydrochlorid
Verwendetes Bromderivat: 4-Brom-o-xylol
Ausbeute: 0,048 g (32% der Theorie)
ESI-MS: 214 [M+H]⁺ (100)

8. 4-Amino-3',5'-dimethyl-[1,1'-biphenyl]-2-ol-hydrochlorid
Verwendetes Bromderivat: 5-Brom-m-xylol
Ausbeute: 0,021 g (13% der Theorie)
ESI-MS: 214 [M+H]⁺ (100)

9. 4-Amino-2',4',5'-trimethyl-[1,1'-biphenyl]-2-ol-hydrochlorid
Verwendetes Bromderivat: 5-Brom-1,2,4-trimethylbenzol
Ausbeute: 0,023 g (14% der Theorie)
ESI-MS: 228 [M+H]⁺ (100)

10. 4-Amino-4'-chlor [1,1'-biphenyl]-2-ol-hydrochlorid
Verwendetes Bromderivat: 1-Brom-4-chlor-benzol
Ausbeute: 0,016 g (10% der Theorie)
ESI-MS: 220 [M+H]⁺ (100)

11. 4-Amino-3'-chlor-[1,1'-biphenyl]-2-ol-hydrochlorid
Verwendetes Bromderivat: 1-Brom-3-chlor-benzol
Ausbeute: 0,036 g (23% der Theorie)
ESI-MS: 220 [M+H]⁺ (100)

12. 4-Amino-2'-chlor-[1,1'-biphenyl]-2-ol-hydrochlorid
Verwendetes Bromderivat: 1-Brom-2-chlor-benzol
Ausbeute: 0,018 g (12% der Theorie)
ESI-MS: 220 [M+H]⁺ (100)

13. 4-Amino-3',5'-dichlor-[1,1'-biphenyl]-2-ol-hydrochlorid
Verwendetes Bromderivat: 1-Brom-3,5-dichlor-benzol
Ausbeute: 0,074 g (40% der Theorie)
ESI-MS: 254 [M+H]⁺(100)

14. 4-Amino-4'-fluor-[1,1'-biphenyl]-2-ol-hydrochlorid
Verwendetes Bromderivat: 1-Brom-4-fluor-benzol
Ausbeute: 0,047 g (33% der Theorie)
ESI-MS: 204 [M+H]⁺(100)

15. 4-Amino-3',5'-difluor-[1,1'-biphenyl]-2-ol-hydrochlorid
Verwendetes Bromderivat: 1-Brom-4-fluor-benzol
Ausbeute: 0,024 g (16% der Theorie)
ESI-MS: 220 [M-H]⁺(100)

16. 4-Amino-3'-brom-5'-methyl-1,1'-biphenyl-2-ol-hydrochlorid
Verwendetes Bromderivat: 3,5-Dibrom-toluol
Ausbeute: 0,022 g (12% der Theorie)
ESI-MS: 278 [M]⁺ (100)

17. 4-Amino-4'-(trifluormethyl)-[1,1'-biphenyl]-2-ol-hydrochlorid
Verwendetes Bromderivat: 1-Brom-4-(trifluormethyl)-benzol
Ausbeute: 0,017 g (10% der Theorie)
ESI-MS: 254 [M+H]⁺ (100)

18. 4-Amino-3'-(trifluormethyl)-[1,1'-biphenyl]-2-ol-hydrochlorid
Verwendetes Bromderivat: 1-Brom-4-(trifluormethyl)-benzol
Ausbeute: 0,017 g (10% der Theorie)
ESI-MS: 254 [M+H]⁺(100)

19. 4-Amino-3'-nitro-[1,1'-biphenyl]-2-ol-hydrochlorid
Verwendetes Bromderivat: 1-Brom-3-nitro-benzol
Ausbeute: 0,029 g (18% der Theorie)
ESI-MS: 253 [M+Na]⁺ (100)
¹H-NMR (300 MHz, DMSO): δ = 10,40 (s, br, 1H, OH); 8,16 (d, J=8,0 1H, H4'); 8,00 (d, J=8,0, 1H, H6'); 7,72 (dd, J=8,0, J=8,0, 1H, H5'); 7,43 (d, J=8,2, 1H, H6); 6,92 (s, 1H, H3); 6,78 (d, J=8,2, 1H, H5); 3,46 (s, br, 3H, NH3⁺).

20. 4-Amino-4'-methyl-3'-nitro[1,1'-biphenyl]-2-ol-hydrochlorid
Verwendetes Bromderivat: 4-Brom-2-nitrotoluol
Ausbeute: 0,082 g (49% der Theorie)
ESI-MS: 267 [M+Na]⁺ (100)
¹H-NMR (300 MHz, DMSO): δ = 10,39 (s, br, 1H, OH); 8,16 (d, J=1,7, 1H, H2'); 7,81 (dd, J=1,7, J=8,0, 1H, H6'); 7,54 (d, J=8,0, 1H, H5'); 7,41 (d, J=8,2, 1H, H6); 6,98 (d, J=1,8, 1H, H3); 6,82 (dd, J=1,8, J=8,1, 1H, H5); 3,46 (s, br, 3H, NH3⁺); 2,54 (s, 3H, CH3).

| CHN-Analyse: | | | | | |
|---|---|---|---|---|---|
| (C₁₃H₁₃N₂O₃HCl) | | % C | % H | % N | %Cl |
| | berechnet: | 55,62 | 4.67 | 9,98 | 12,63 |
| | gefunden: | 55,10 | 4,60 | 9,50 | 12,20 |

21. 4-Amino-2'-nitro-4'-(trifluormethyl)[1,1'-biphenyl]-2-ol-hydrochlorid
Verwendetes Bromderivat: 1-Brom-2-nitro-4-(trifluormethyl)-benzol
Ausbeute: 0,057 g (28% der Theorie)
ESI-MS: 297 [M-H]⁺ (100)

22. 4'-Amino-2'-hydroxy-[1,1'-biphenyl]-3-carbonitril-hydrochlorid
Verwendetes Bromderivat: 3-Brom-benzonitril
Ausbeute: 0,036 g (24% der Theorie)
ESI-MS: 233 [M+Na]⁺(100)

23. 4-Amino-4'-methoxy-[1,1'-biphenyl]-2-ol-hydrochlorid
Verwendetes Bromderivat: 1-Brom-4-methoxy-benzol
Ausbeute: 0,021 g (14% der Theorie)
ESI-MS: 216 [M+H]⁺(100)

24. 4-Amino-3'-methoxy-[1,1'-biphenyl]-2-ol hydrochlorid
Verwendetes Bromderivat: 1-Brom-3-methoxy-benzol
Ausbeute: 0.01 g (7% der Theorie)
ESI-MS: 216 [M+H]⁺ (100)

25. 4-Amino-2'-methoxy-[1,1'-biphenyl]-2-ol-hydrochlorid
Verwendetes Bromderivat: 1-Brom-2-methoxy-benzol
Ausbeute: 0,058 g (36% der Theorie)
ESI-MS: 214 [M-H]⁺ (100)

26. 4-Amino-4'-ethoxy-[1,1'-biphenyl]-2-ol-hydrochlorid
Verwendetes Bromderivat: 1-Brom-4-ethoxy-benzol
Ausbeute: 0,020 g (13% der Theorie)
ESI-MS: 230 [M+H]⁺(100)

27. 4-Amino-2',4'-dimethoxy-[1,1'-biphenyl]-2-ol-hydrochlorid
Verwendetes Bromderivat: 1-Brom-2,4-dimethoxy-benzol
Ausbeute: 0,050 g (30% der Theorie)
ESI-MS: 268 [M+Na]⁺(100)

28. 4-Amino-2',5'-dimethoxy-[1,1'-biphenyl]-2-ol-hydrochlorid
Verwendetes Bromderivat: 2-Brom-1,4-dimethoxy-benzol
Ausbeute: 0,061 g (36% der Theorie)
ESI-MS: 268 [M+Na]⁺ (100)

29. 5-Amino-2-(1,3-benzodioxol-5-yl)-phenol-hydrochlorid
Verwendetes Bromderivat: 5-Brom-1,3-benzodioxol
Ausbeute: 0,030 g (19% der Theorie)
ESI-MS: 230 [M+H]⁺ (100)
¹H-NMR (300 MHz, DMSO): δ = 10,10 (s, br, 1H, OH); 7,28 (d, J=8,2, 1H, H3); 7,09 (s, 1H, H4'); 6,96 (m, 2H, H6' und H7'); 6,91 (s, 1H, H6); 6,77 (d, J=8,2, 1H, H4); 6,04 (s, 2H, CH2O); 3,50 (s, br, 3H, NH3⁺).

30. 4-Amino-4'-methoxy-2'-methyl-[1,1'-biphenyl]-2-ol-hydrochlorid
Verwendetes Bromderivat: 1-Brom-4-methoxy-2-methyl-benzol
Ausbeute: 0,016 g (10% der Theorie)
ESI-MS: 230 [M+H]⁺ (100)

31. 4-Amino-4'-phenoxy-[1,1'-biphenyl]-2-ol-hydrochlorid
Verwendetes Bromderivat: 1-Brom-4-phenoxy-benzol
Ausbeute: 0,024 g (13% der Theorie)
ESI-MS: 276 [M-H]⁺ (100)

32. 4-Amino-[1,1'-biphenyl]-2,4'-diol-hydrochlorid
Verwendetes Bromderivat: 4-Brom-phenol
Ausbeute: 0,033 g (23% der Theorie)
ESI-MS: 200 [M-H]⁺(100)

33 4-Amino-2'-methyl-[1,1'-biphenyl]-2,4'-diol-hydrochlorid
Verwendetes Bromderivat: 4-Brom-3-methyl-phenol
Ausbeute: 0,012 g (8% der Theorie)
ESl-MS: 216 [M+H]⁺ (100)

34. 3',4-Diamino-[1,1'-biphenyl]-2-ol-hydrochlorid
Verwendetes Bromderivat: 3-Brom-anilin
Ausbeute: 0,032 g (23% der Theorie)
ESI-MS: 199 [M-H]⁺ (100)

35. 1-(4'-Amino-2'-hydroxy-1,1'-biphenyl-4-yl)ethanon-hydrochlorid
Verwendetes Bromderivat: 4-Bromacetophenon
Ausbeute: 0,016 g (10% der Theorie)
ESI-MS: 250 [M+Na]⁺ (100)

36. 4-Amino-1,1':3',1"-terphenyl-2-ol hydrochlorid
Verwendetes Bromderivat: 3-Brom-1,1'-biphenyl
Ausbeute: 0,050 g (28% der Theorie)
ESI-MS: 260 [M-H]⁺ (100)

37. 5-Amino-2-(3-pyridinyl)-phenol-hydrochlorid
Verwendetes Bromderivat: 3-Brom-pyridin
Ausbeute: 0,067 g (50% der Theorie)
ESI-MS: 187 [M+H]⁺ (100)
¹H-NMR (300 MHz, DMSO): δ = 10,88 (s, br, 1H, OH); 9,09 (s, 1H, H2'); 8,81 (d, J=5,5, 1H, H4'); 8,76 (d, J=8,2, 1H, H6'); 8,09 (dd, J=5,5, J=8,2, 1H, H5'); 7,54 (d, J=8,3, 1H, H3); 7,02 (d, J=1,7, 1H, H6); 6,84 (dd, J=1,7, J=8,3, 1H, H4); 4,2 (s, br, 3H, NH3⁺).

38. 5-Amino-2-(2-pyridinyl)-phenol-hydrochlorid
Verwendetes Bromderivat: 2-Brom-pyridin
Ausbeute: 0,038 g (28% der Theorie)
ESI-MS: 187 [M+H]⁺ (100)

39. 5-Amino-2-(3-methyl-2-pyridinyl)-phenol-hydrochlorid
Verwendetes Bromderivat: 2-Brom-3-methyl-pyridin
Ausbeute: 0,039 g (27% der Theorie)
ESI-MS: 201 [M+H]⁺ (100)

40. 5-Amino-2-(4-methyl-2-pyridinyl)-phenol-hydrochlorid
Verwendetes Bromderivat: 2-Brom-4-methyl-pyridin
Ausbeute: 0,057 g (39% der Theorie)
ESI-MS: 201 [M+H]⁺ (100)

41. 5-Amino-2-(5-methyl-2-pyridinyl)-phenol-hydrochlorid
Verwendetes Bromderivat: 2-Brom-5-methyl-pyridin
Ausbeute: 0,051 g (35% der Theorie)
ESI-MS: 201 [M+H]⁺(100)

42. 5-Amino-2-(6-methyl-2-pyridinyl)-phenol-hydrochlorid
Verwendetes Bromderivat: 2-Brom-6-methyl-pyridin
Ausbeute: 0,056 g (39% der Theorie)
ESI-MS: 201 [M+H]⁺ (100)

43. 5-Amino-2-(5-nitro-2-pyridinyl)-phenol-hydrochlorid
Verwendetes Bromderivat: 2-Brom-5-nitro-pyridin
Ausbeute: 0,060 g (37% der Theorie)
ESI-MS: 230 [M-H]⁺ (100)

44. 5-Amino-2-(5-bromo-3-pyridinyl)-phenol-hydrochlorid
Verwendetes Bromderivat: 3,5-Dibrom-pyridin
Ausbeute: 0,047 g (26% der Theorie)
ESI-MS: 265 [M]⁺ (100)
¹H-NMR (300 MHz, DMSO): δ = 10,54 (s, br, 1H, OH); 8,75 (s, 1H, H2'); 8,67 (s, 1H, H6'); 8,24 (s, 1H, H4'); 7,45 (d, J=8,2, 1H, H3); 6,97 (s, 1H, H6); 6,82 (d, J=8,2, 1H, H4).

45. 5-Amino-2-(5-pyrimidinyl)-phenol-hydrochlorid
Verwendetes Bromderivat: 5-Brom-pyrimidin
Ausbeute: 0,067 g (50% der Theorie)
ESI-MS: 188 [M+H]⁺(100)
¹H-NMR (300 MHz, DMSO): δ = 10,74 (s, br, 1H, OH); 9,14 (s, 1H, H2'); 9,00 (s, 2H, H4' und H6'); 7,52 (d, J=8,1, 1H, H3); 7,10 (d, J=1,6, 1H, H6); 6,93 (dd, J=1,6, J=8,1, 1H, H4); 3,76 (s, br, 3H, NH3⁺).

### Beispiele 46 bis 90: Haarfärbemittel

Es werden Haarfärbelösungen der folgenden Zusammensetzung hergestellt:

| | |
|---|---|
| 1,25 mmol | Substanz der Formel (I) gemäß Tabelle 1 |
| 1,25 mmol | Entwicklersubstanz gemäß Tabelle 1 |
| 10,0 g | Laurylethersulfat (28prozentige wässrige Lösung) |
| 9,0 g | Ammoniak (22prozentige wässrige Lösung) |
| 7,8 g | Ethanol |
| 0,3 g | Ascorbinsäure |
| 0,3 g | Ethylendiaminotetraessigsäure-Dinatriumsalz-Hydrat |
| ad 100,0 g | Wasser, vollentsalzt |

10 g der vorstehenden Färbelösung werden unmittelbar vor der Anwendung mit 10 g einer 6prozentigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die resultierenden Färbungen sind in Tabelle 1 zusammengefasst.

**Tabelle 1:**

| **Beispiel Nr.** | **Kupplersubstanz der Formel (I)** | **Entwicklersubstanz** | | | |
|---|---|---|---|---|---|
| | | **I. 2,5-Diaminotoluolsulfat** | **II. 2,5-Diaminophenylethanolsulfat** | **III. 4,5-Diamino-1-(2'-hydroxyethyl)-pyrazol-sulfat** | **IV. 4-Amino-3-methylphenol** |
| **46.** | gemäß Beispiel **1** | violett | violett | purpur | hell-rosa |
| **47.** | gemäß Beispiel **2** | violett | violett | purpur | hell-rosa |
| **48.** | gemäß Beispiel **3** | dunkelviolett | dunkelviolett | purpur | hell-rosa |
| **49.** | gemäß Beispiel **4** | aschblond | aschblond | hellrot | beige |
| **50.** | gemäß Beispiel **5** | hellgrauviolett | hellgrauviolett | hellrot | beige |
| **51.** | gemäß Beispiel **6** | violett | violett | hellrot | beige |
| **52.** | gemäß Beispiel **7** | violett | violett | hellrot | hell-rosa |
| **53.** | gemäß Beispiel **8** | violett | violett | purpur | hell-rosa |
| **54.** | gemäß Beispiel **9** | grau | grau | hellrot | beige |
| **55.** | gemäß Beispiel **10** | violett | violett | purpur | hell-rosa |
| **56**. | gemäß Beispiel **11** | violett | violett | purpur | hell-rosa |
| **57.** | gemäß Beispiel **12** | grauviolett | grauviolett | purpur | beige |
| **58.** | gemäß Beispiel **13** | graublau | graublau | hellrot | beige |
| **59.** | gemäß Beispiel **14** | violett | violett | hellrot | beige |
| **60**. | gemäß Beispiel **15** | blauviolett | blauviolett | himbeerrot | hell-rosa |
| **61.** | gemäß Beispiel **16** | hell-violett | hell-violett | hell-rot | beige |
| **62**. | gemäß Beispiel **17** | hell-grauviolett | hell-grauviolett | hellrot | beige |
| **63.** | gemäß Beispiel **18** | blaustichiges violett | blaustichiges violett | himbeerrot | hell-rosa |
| **64.** | gemäß Beispiel **19** | dunkelblaustichiges violett | dunkelblaustichiges violett | himbeerrot | hell-rosa |
| **65.** | gemäß Beispiel **20** | dunkelblaustichiges violett | dunkelblaustichiges violett | himbeerrot | hell-rosa |
| **66**. | gemäß Beispiel **21** | aschblond | aschblond | hellrot | beige |
| **67**. | gemäß Beispiel **22** | blaustichiges violett | blaustichiges violett | himbeerrot | hell-rosa |
| **68**. | gemäß Beispiel **23** | violett | violett | hellrot | beige |
| **69**. | gemäß Beispiel **24** | dunkel-violett | dunkel-violett | purpur | hell-rosa |
| **70**. | gemäß Beispiel **25** | violett | violett | hellrot | beige |
| **71.** | gemäß Beispiel **26** | hellviolett | hellviolett | hellrot | beige |
| **72**. | gemäß Beispiel **27** | hell-grauviolett | hell-grauviolett | hellrot | beige |
| **73**. | gemäß Beispiel **28** | aschblond | aschblond | hellrot | beige |
| **74**. | gemäß Beispiel **29** | violett | violett | purpur | hellrosa |
| **75**. | gemäß Beispiel **30** | hellviolett | hellviolett | hellrot | beige |
| **76**. | gemäß Beispiel **31** | hellviolett | hellviolett | hellrot | beige |
| **77.** | gemäß Beispiel **32** | dunkel-violett | dunkel-violett | purpur | hell-ro: |
| **78**. | gemäß Beispiel **33** | hellviolett | hellviolett | hellrot | beige |
| **79**. | gemäß Beispiel **34** | dunkel-violett | dunkel-violett | purpur | hell-rosa |
| **80**. | gemäß Beispiel **35** | hellblaustichiges violett | hellblaustichiges violett | hellhimbeerrot | hell-rosa |
| **81**. | gemäß Beispiel **36** | hell-violett | hell-violett | hellrot | beige |
| **82**. | gemäß Beispiel **37** | dunkelblau | dunkelblau | himbeerrot | hell-rosa |
| **83**. | gemäß Beispiel **38** | aschblond | aschblond | hellrot | beige |
| **84**. | gemäß Beispiel **39** | aschblond | aschblond | hellrot | beige |
| **85**. | gemäß Beispiel **40** | aschblond | aschblond | hellrot | beige |
| **86**. | gemäß Beispiel **41** | grau | grau | hellrot | beige |
| **87**. | gemäß Beispiel **42** | grau | grau | hellrot | beige |
| **88**. | gemäß Beispiel **43** | hell-blond | hell-blond | hellrot | gelb |
| **89**. | gemäß Beispiel **44** | dunkelblau | dunkelblau | himbeerrot | hell-rosa |
| **90**. | gemäß Beispiel **45** | dunkelblau | dunkelblau | himbeerrot | hell-ro |

### Beispiel 91 bis 114: Haarfärbemittel

Es werden Haarfärbelösungen der folgenden Zusammensetzung hergestellt:

| | |
|---|---|
| X g | 3-Aminophenol-Derivat der Formel (I) |
| | (Kupplersubstanz **K1** bis **K4** gemäss Tabelle 4) |
| U g | Entwicklersubstanz **E8** bis **E15** gemäß Tabelle 2 |
| Y g | Kupplersubstanz **K12** bis **K36** gemäß Tabelle 4 |
| 10,0 g | Laurylethersulfat (28prozentige wässrige Lösung) |
| 9,0 g | Ammoniak (22prozentige wässrige Lösung) |
| 7,8 g | Ethanol |
| 0,3 g | Ascorbinsäure |
| 0,3 g | Ethylendiaminotetraessigsäure-Dinatriumsalz-Hydrat |
| ad 100,0 g | Wasser, vollentsalzt |

30 g der vorstehenden Färbelösung werden unmittelbar vor der Anwendung mit 30 g einer 6prozentigen wässrigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die Färbeergebnisse sind in Tabelle 5 zusammengefasst.

**Tabelle 2:**

| **Entwicklersubstanzen** | |
|---|---|
| **E8** | 1,4-Diaminobenzol |
| **E9** | 2,5-Diamino-phenylethanol-sulfat |
| **E10** | 3-Methyl-4-amino-phenol |
| **E11** | 4-Amino-2-aminomethyl-phenol-dihydrochlorid |
| **E13** | N,N-Bis(2'-hydroxyethyl)-p-phenylendiamin-sulfat |
| **E14** | 4,5-Diamino-1-(2'-hydroxyethyl)-pyrazol-sulfat |
| **E15** | 2,5-Diaminotoluol-sulfat |

**Tabelle 3:**

| **Direktziehende Farbstoffe** | |
|---|---|
| **D2** | 6-Chlor-2-ethylamino-4-nitro-phenol |
| **D3** | 2-Amino-6-chlor-4-nitro-phenol |

**Tabelle 4:**

| **Kupplersubstanzen** | |
|---|---|
| **K1** | 4-Amino-3'-methyl-[1,1'-biphenyl]-2-ol |
| **K2** | 4-Amino-4'-methyl-3'-nitro[1,1'-biphenyl]-2-ol |
| **K3** | 5-Amino-2-(3-pyridinyl)-phenol |
| **K4** | 5-Amino-2-(5-pyrimidinyl)-phenol |
| | |
| **K12** | 2-Amino-4-(2'-hydroxyethyl)amino-anisol-sulfat |
| **K13** | 1,3-Diamino-4-(2'-hydroxyethoxy)benzol-sulfat |
| **K14** | 2,4-Diamino-5-fluor-toluol-sulfat |
| **K18** | N-(3-Dimethylamino)phenylharnstoff |
| **K19** | 1,3-Bis(2,4-diaminophenoxy)propan-tetrahydrochlorid |
| **K21** | 3-Amino-phenol |
| **K22** | 5-Amino-2-methyl-phenol |
| **K23** | 3-Amino-2-chlor-6-methyl-phenol |
| **K24** | 5-Amino-4-fluor-2-methyl-phenol-sulfat |
| **K25** | 1-Naphthol |
| **K31** | 1,3-Dihydroxy-benzol |
| **K32** | 2-Methyl-1,3-dihydroxy-benzol |
| **K33** | 1-Chlor-2,4-dihydroxy-benzol |
| **K34** | 4-(2'-Hydroxyethyl)amino-1,2-methylendioxybenzolhydrochlorid |
| **K36** | 2-Amino-5-methyl-phenol |

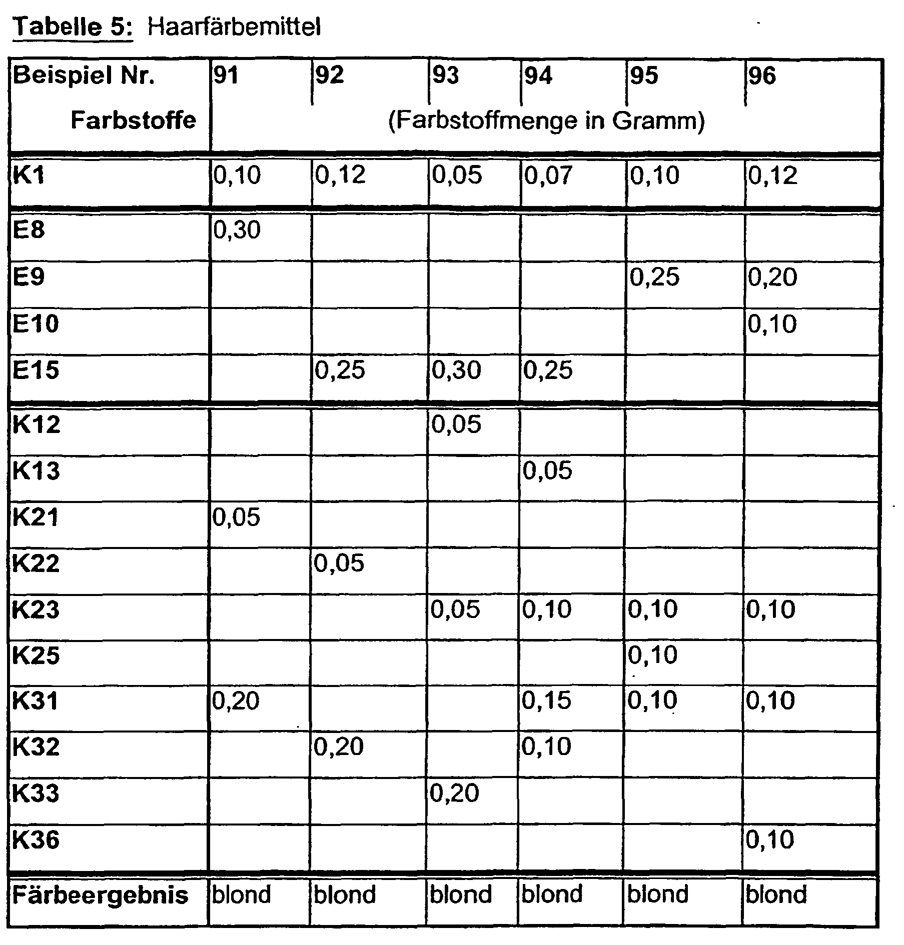

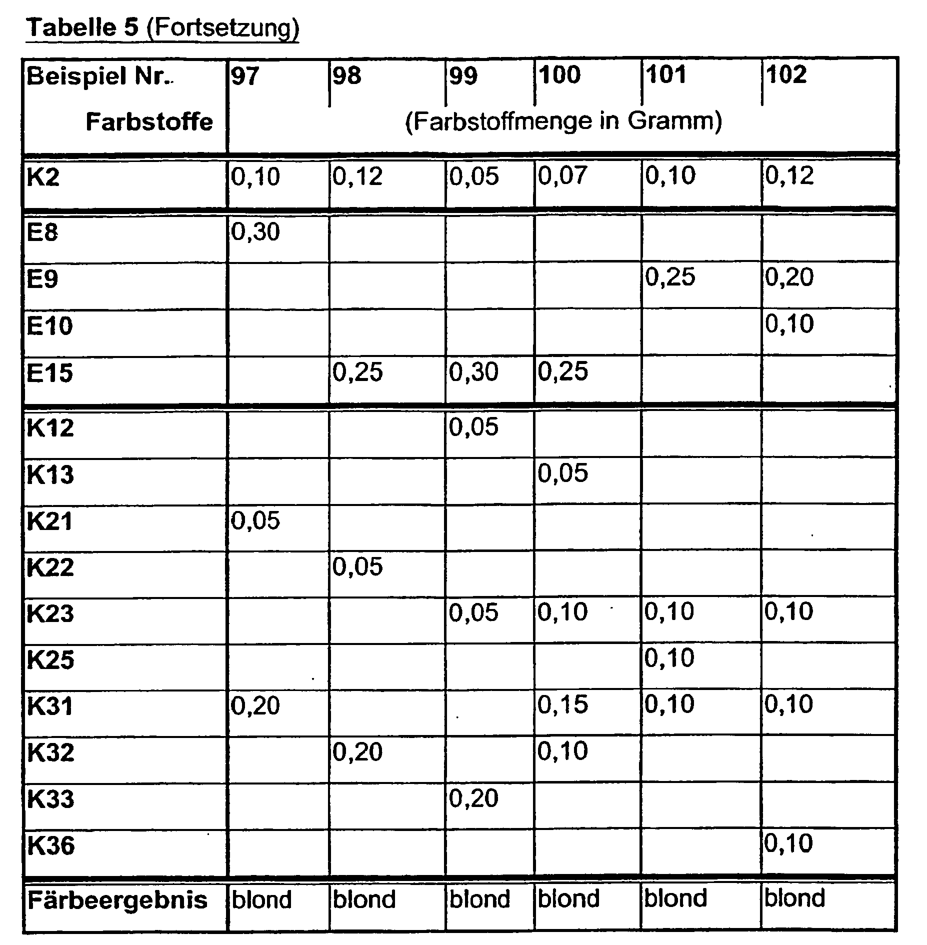

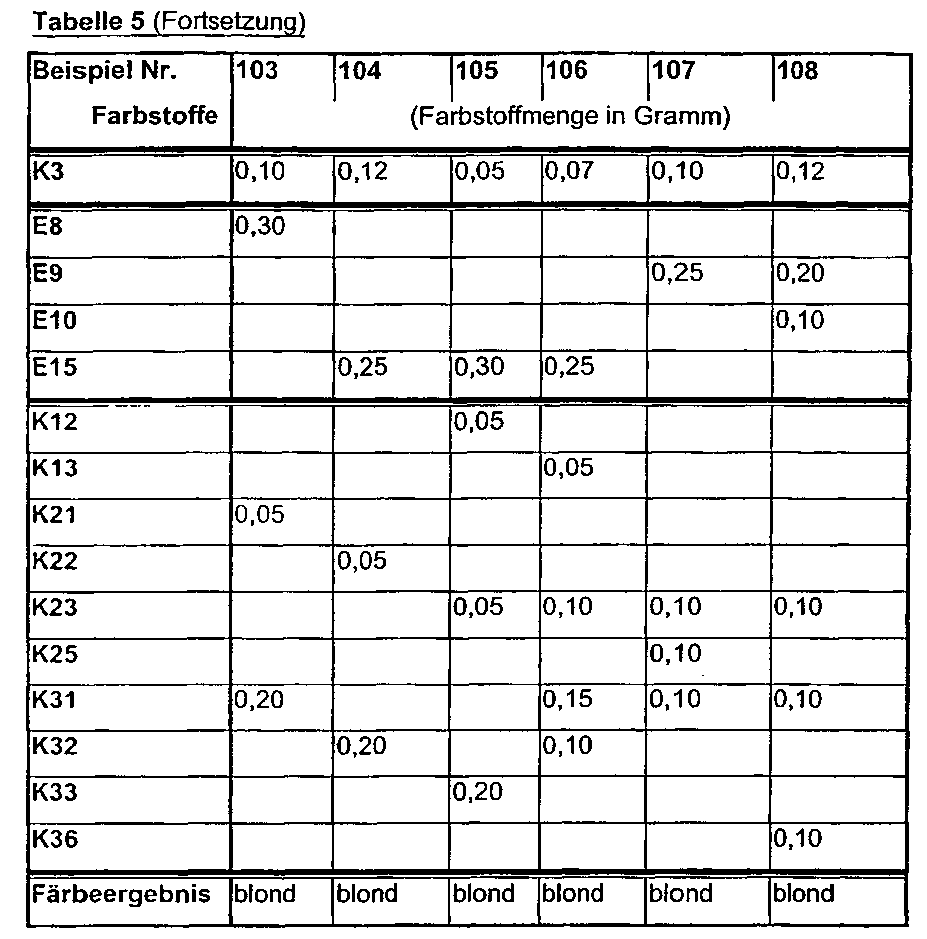

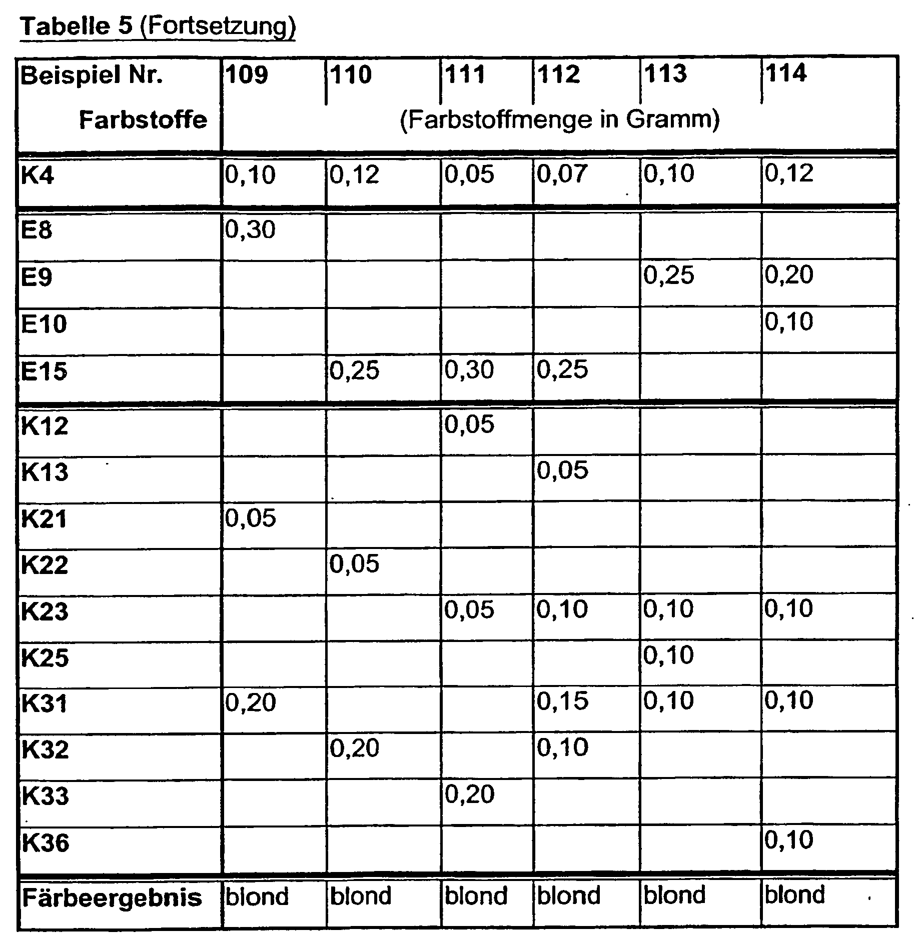

### Beispiele 115 bis 138: Haarfärbemittel

Es werden Haarfärbelösungen der folgenden Zusammensetzung hergestellt:

| | |
|---|---|
| X g | 3-Aminophenol-Derivat der Formel (I) |
| | (Kupplersubstanz **K1** bis **K4** gemäss Tabelle 4) |
| U g | Entwicklersubstanz **E8** bis **E15** gemäß Tabelle 2 |
| Y g | Kupplersubstanz **K12** bis **K36** gemäß Tabelle 4 |
| Z g | direktziehende Farbstoffe **D2** und/oder **D3** gemäß Tabelle 3 |
| 10,0 g | Laurylethersulfat (28prozentige wässrige Lösung) |
| 9,0 g | Ammoniak (22prozentige wässrige Lösung) |
| 7,8 g | Ethanol |
| 0,3 g | Ascorbinsäure |
| 0,3 g | Ethylendiaminotetraessigsäure-Dinatriumsalz-Hydrat |
| ad 100,0 g | Wasser, vollentsalzt |

30 g der vorstehenden Färbecreme werden unmittelbar vor der Anwendung mit 30 g einer 6prozentigen Wasserstoffperoxidlösung vermischt. Anschliessend wird das Gemisch auf das Haar aufgetragen. Nach einer Einwirkzeit von 30 Minuten bei 40 °C wird das Haar mit Wasser gespült, mit einem handelsüblichen Shampoo gewaschen und getrocknet. Die Färbeergebnisse sind in Tabelle 6 zusammengefasst.

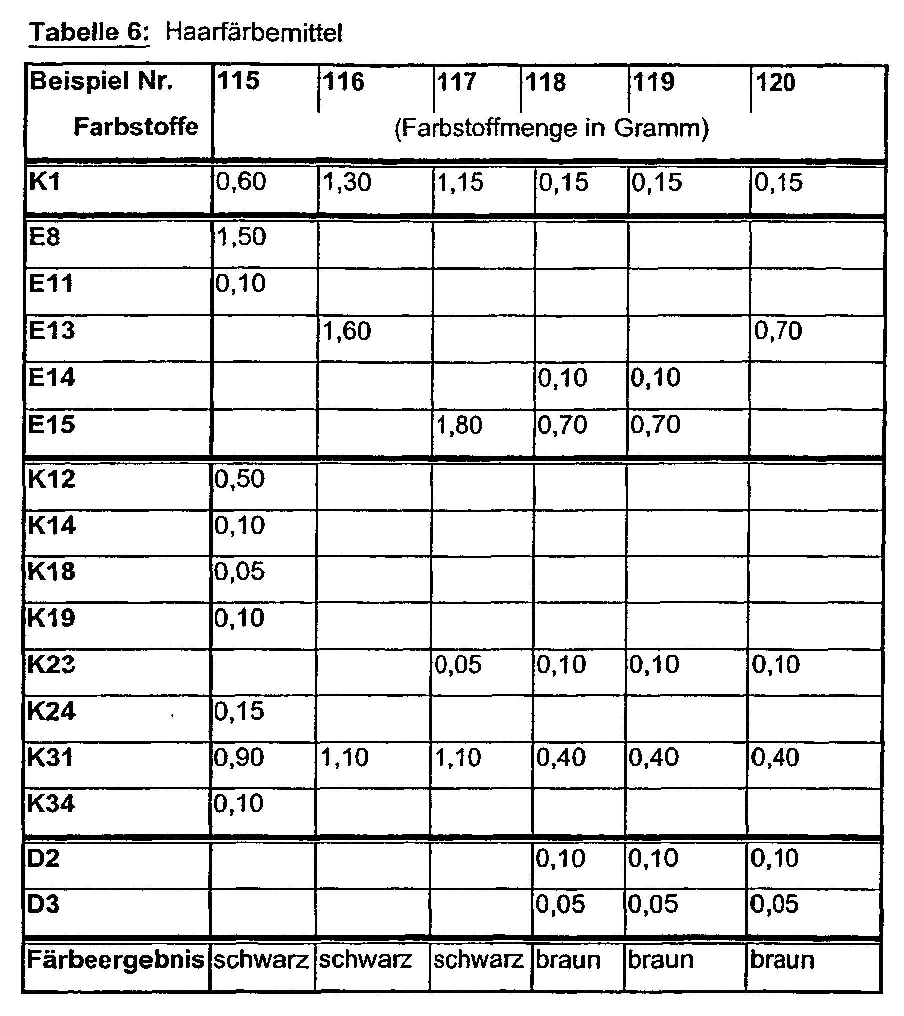

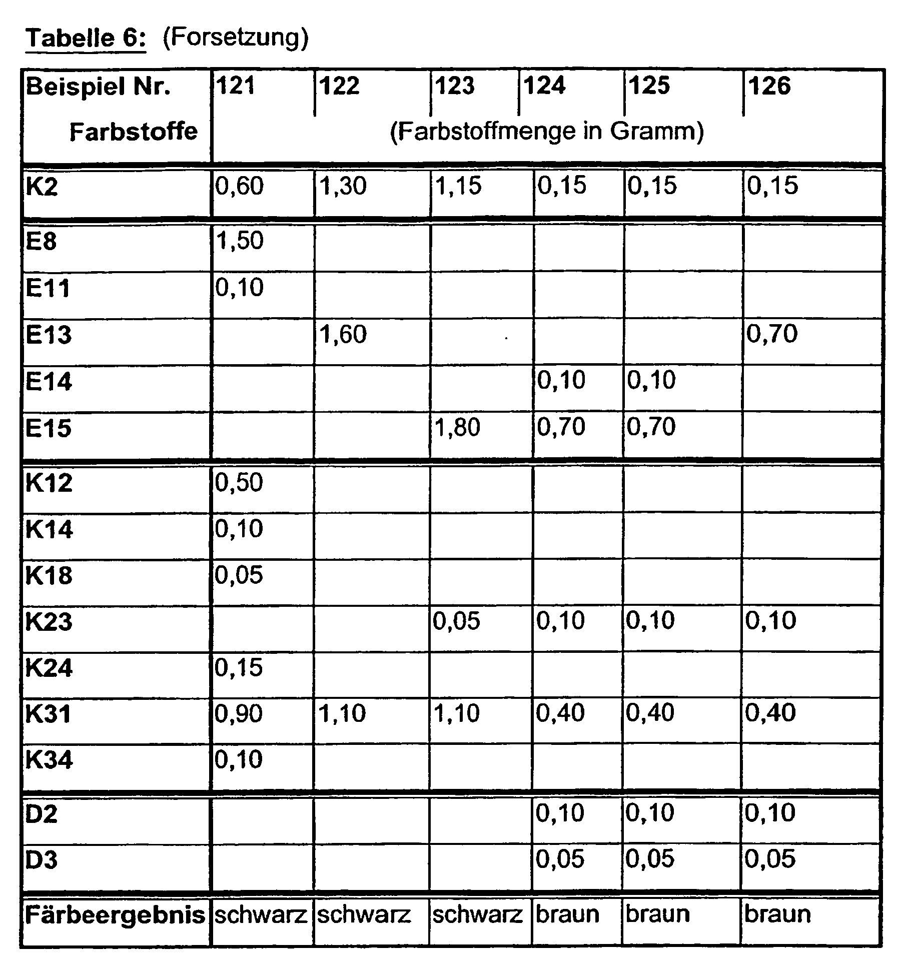

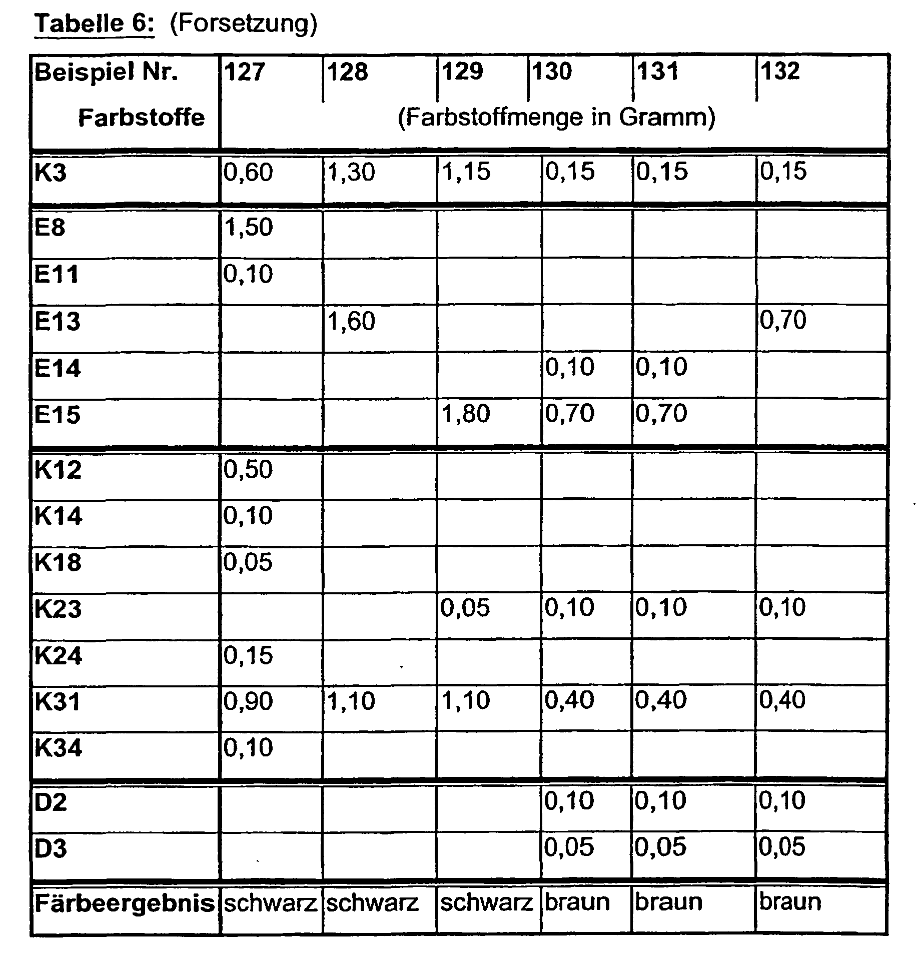

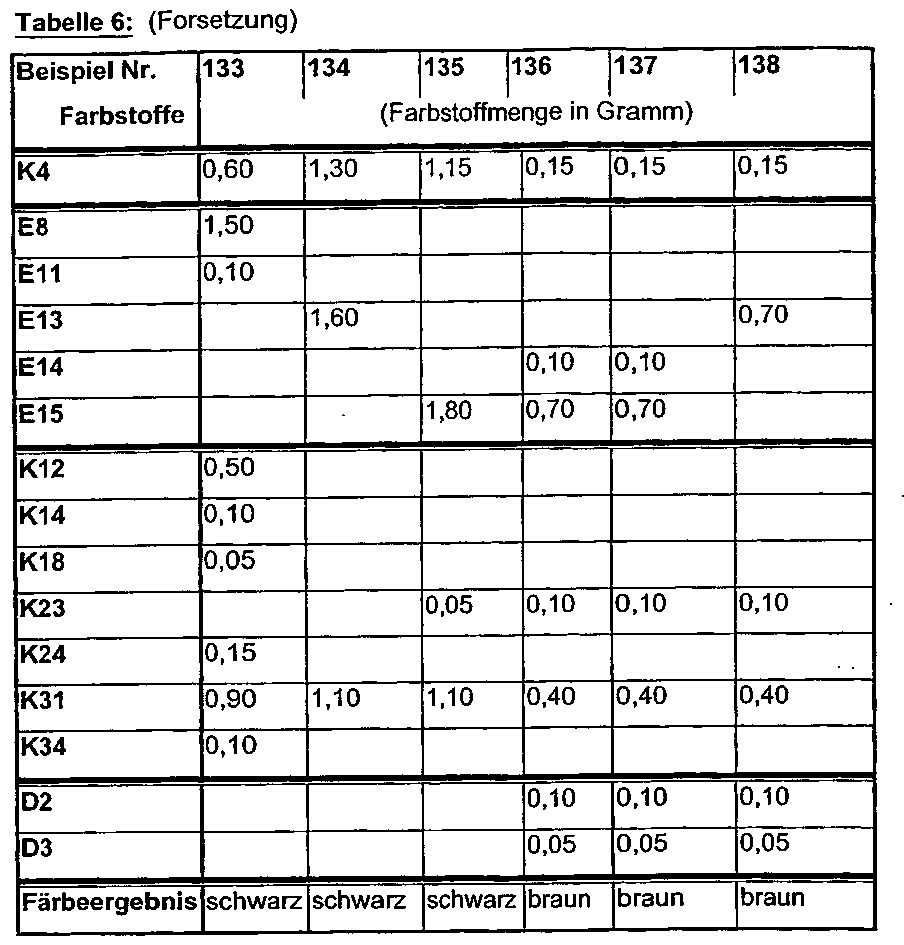

Alle in der vorliegenden Anmeldung enthaltenen Prozentangaben stellen soweit nicht anders angegeben Gewichtsprozente dar.

## Patentansprüche

1. Mittel zur Färbung von Keratinfasem auf der Basis einer Entwicklersubstanz-Kupplersubstanz-Kombination, **dadurch gekennzeichnet, dass** es mindestens ein 3-Aminophenol-Derivat der Formel (I) oder dessen physiologisch verträgliches, wasserlösliches Salz enthält, 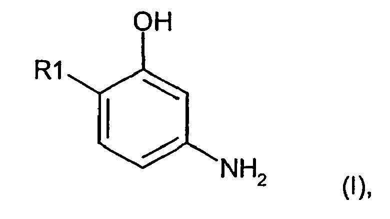 worin
**R1** gleich einem Rest der Formel (II) oder (III) ist, 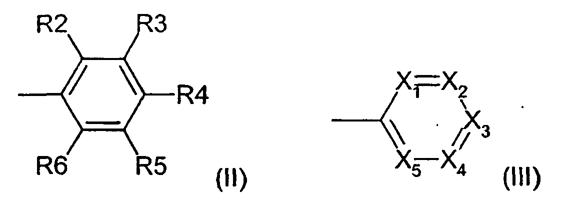 wobei **R2, R3, R4, R5** und **R6** unabhängig voneinander Wasserstoff, ein Halogenatom, eine Cyanogruppe, eine Hydroxygruppe, eine C₁-C₄-Alkoxygruppe, eine Phenoxygruppe, eine C₁-C₄-Hydroxyalkoxygruppe, eine C₁-C₆-Alkylgruppe, eine Phenylgruppe, eine C₁-C₄-Alkylthioethergruppe, eine Mercaptogruppe, eine Nitrogruppe, eine Aminogruppe, eine C₁-C₄-Alkylaminogruppe, eine Hydroxy(C₂-C₄)alkylaminogruppe, eine Di(C₁-C₄)alkylaminogruppe, eine Di(hydroxy(C₂-C₄)alkyl)-aminogruppe, eine (Dihydroxy(C₃-C₄)alkyl)-aminogruppe, eine (Hydroxy(C₂-C₄)alkyl)-C₁-C₄-alkylaminogruppe, eine Trifluormethangruppe eine -C(O)H-Gruppe, eine -C(O)CH₃-Gruppe, eine -C(O)CF₃-Gruppe, eine -Si(CH₃)₃-Gruppe, eine (C₁-C₄)-Hydroxyalkylgruppe, eine (C₂-C₄)-Dihydroxyalkylgruppe, eine (C₁-C₄)-Aminoalkylgruppe, oder eine (C₁-C₄)-Cyanoalkylgruppe darstellen, oder zwei nebeneinanderliegende Reste R2 bis R6 eine -O-CH2-O-Brücke bilden;
**X**_{**1**}**, X**_{**2**}**, X**_{**3**}**, X**_{**4**} und **X**_{**5**} unabhängig voneinander gleich Stickstoff oder einer C-R7-Gruppe, C-R8-Gruppe, C-R9-Gruppe, C-R10-Gruppe oder C-R11-Gruppe sind, unter der Bedingung, dass mindestens einer und höchstens drei der Reste **X1** bis **X5** Stickstoff bedeuten; und
**R7, R8, R9, R10** und **R11** unabhängig voneinander Wasserstoff, ein Halogenatom, eine Cyanogruppe, eine C₁-C₆-Alkylgruppe, eine (C₁-C₄)-Alkylthioethergruppe, eine Mercaptogruppe, eine Nitrogruppe, eine Aminogruppe, eine (C₁-C₄)-Alkylaminogruppe, eine Di(C₁-C₄)alkylaminogruppe, eine Trifluormethangruppe, eine -C(O)H-Gruppe, eine -C(O)CH₃-Gruppe, eine -C(O)CF₃-Gruppe, eine -Si(CH₃)₃-Gruppe, eine -C(O)-NH₂ Gruppe, eine (C₁-C₄)-Hydroxyalkylgruppe oder eine (C₂-C₄)-Dihydroxyalkylgruppe darstellen.

2. Mittel nach Anspruch 1, **dadurch gekennzeichnet, dass** für die Verbindungen der Formel (I) gilt: (i) **R1** ist gleich einem Rest der Formel (II) mit **R2** und **R6** gleich Wasserstoff oder (ii) **R1** ist gleich einem Rest der Formel (III) mit **X1** und **X5** gleich **C-R7** und **C-R11**, wobei **R7** und **R11** gleich Wasserstoff sind.

3. Mittel nach Anspruch 1 oder 2, **dadurch gekennzeichnet, dass** die Verbindungen der Formel (I) ausgewählt sind aus 4-Amino-3'-methyl-[1,1'-biphenyl]-2-ol, 4-Amino-4'-methyl-3'-nitro[1,1'-biphenyl]-2-ol, 4-Amino-[1,1'-biphenyl]-2,4'-diol, 5-Amino-2-(3-pyridinyl)-phenol und 5-Amino-2-(5-pyrimidinyl)-phenol sowie deren physiologisch verträglichen wasserlöslichen Salzen.

4. Mittel nach einem der Ansprüche 1 bis 3 **dadurch gekennzeichnet, dass** das 3-Aminophenol-Derivat der Formel (I) in einer Menge von 0,005 bis 20 Gewichtsprozent enthalten ist.

5. Mittel nach einem der Ansprüche 1 bis 4, **dadurch gekennzeichnet, dass** die Entwicklersubstanz ausgewählt ist aus der Gruppe bestehend aus 1,4-Diamino-benzol, 1,4-Diamino-2-methyl-benzol, 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diamino-biphenyl, 1,4-Diamino-2-methoxymethyt-benzol, 1,4-Diamino-2-amino-methyl-benzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)amino]-anilin, 4-[Di(2-hydroxyethyl)amino]-anilin, 4-[Di(2-hydroxyethyl)amino]-2-methyl-anilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(1-hydroxyethyl)-benzol, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluor-phenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-aminolmethylphenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1H-pyrazol, 4,5-Diamino-1-[(4-methylphenyl)methyl]-1H-pyrazol, 1-[(4-Chlorphenyl)methyl]-4,5-diamino-1H-pyrazol, 4,5-Diamino-1-methyl-1H-pyrazol, 2-Amino-phenol, 2-Amino-6-methyl-phenol, 2-Amino-5-methyl-phenol und 1,2,4-Trihydroxy-benzol.

6. Mittel nach einem der Ansprüche 1 bis 5, **dadurch gekennzeichnet, dass** es zusätzlich zu den Verbindungen der Formel (I) mindestens eine weitere bekannte Kupplersubstanzen enthält, welche ausgewählt ist aus der Gruppe bestehend aus N-(3-Dimethylamino-phenyl)-harnstoff, 2,6-Diamino-pyridin, 2-Amino-4-[(2-hydroxyethyl)amino]-anisol, 2,4-Diamino-1-fluor 5-methyl-benzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1 -(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlorphenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion.

7. Mittel nach einem der Ansprüche 1 bis 6 , **dadurch gekennzeichnet, dass** die Entwicklersubstanzen und Kupplersubstanzen, bezogen auf die Gesamtmenge des Färbemittels, jeweils in einer Gesamtmenge von 0,005 bis 20 Gewichtsprozent enthalten sind.

8. Mittel nach einem der Ansprüche 1 bis 7 , **dadurch gekennzeichnet, dass** es zusätzlich mindestens einen direktziehenden Farbstoff enthält.

9. Mittel nach einem der Ansprüche 1 bis 8, **dadurch gekennzeichnet, dass** es einen pH-Wert von 6,5 bis 11,5 aufweist.

10. Gebrauchsfertiges Mittel zur oxidativen Färbung von Keratinfasern, welche in einem zum Färben geeigneten Medium mindestens eine Entwicklersubstanz und mindestens eine Kupplersubstanz sowie mindestens ein Oxidationsmittel enthält, **dadurch gekennzeichnet, dass** es als Kupplersubstanz mindestens ein 3-Aminophenol-Derivat der Formel (I) gemäß einem der Ansprüche 1 bis 3 enthält.

11. Mittel nach einem der Ansprüche 1 bis 10, **dadurch gekennzeichnet, dass** es ein Haarfärbemittel ist.

12. 3-Aminophenol-Derivat der Formel (I) oder dessen physiologisch verträgliches, wasserlösliches Salz, 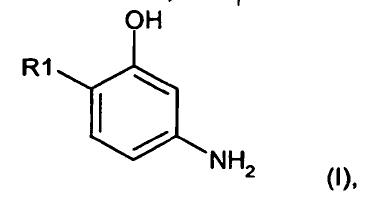 worin
**R1** gleich einem Rest der Formel (II) oder (III) ist, 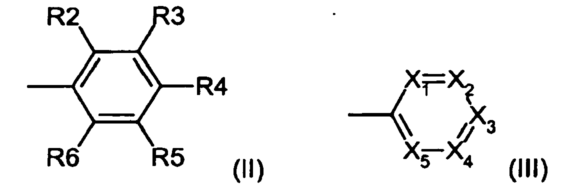 wobei **R2, R3, R4, R5** und **R6** unabhängig voneinander Wasserstoff, ein Halogenatom, eine Cyanogruppe, eine Hydroxygruppe, eine C₁-C₄-Alkoxygruppe, eine Phenoxygruppe, eine C₁-C₄-Hydroxyalkoxygruppe, eine C₁-C₆-Alkylgruppe, eine Phenylgruppe, eine C₁-C₄-Alkylthioethergruppe, eine Mercaptogruppe, eine Nitrogruppe, eine Aminogruppe, eine C₁-C₄-Alkylaminogruppe, eine Hydroxy(C₂-C₄)alkylaminogruppe, eine Di(C₁-C₄)alkylaminogruppe, eine Di(hydroxy(C₂-C₄)alkyl)-aminogruppe, eine (Dihydroxy(C₃-C₄)alkyl)-aminogruppe, eine (Hydroxy(C₂-C₄)alkyl)-C₁-C₄-alkylaminogruppe, eine Trifluormethangruppe, eine -C(O)H-Gruppe, eine -C(O)CH₃-Gruppe, eine -C(O)CF₃-Gruppe, eine -Si(CH₃)₃-Gruppe, eine (C₁-C₄)-Hydroxyalkylgruppe, eine (C₂-C₄)-Dihydroxyalkylgruppe, eine (C₁-C₄)-Aminoalkylgruppe, oder eine (C₁-C₄)-Cyanoalkylgruppe darstellen, oder zwei nebeneinanderliegende Reste R2 bis R6 eine -O-CH2-O-Brücke bilden;
**X**_{**1**}**, X**_{**2**}**, X**_{**3**}**, X**_{**4**} und **X**_{**5**} unabhängig voneinander gleich Stickstoff oder einer C-R7-Gruppe, C-R8-Gruppe, C-R9-Gruppe, C-R10-Gruppe oder C-R11-Gruppe sind, unter der Bedingung, dass mindestens einer und höchstens drei der Reste **X1** bis **X5** Stickstoff bedeuten; und
**R7, R8, R9, R10** und **R11** unabhängig voneinander Wasserstoff, ein Halogenatom, eine Cyanogruppe, eine C₁-C₆-Alkylgruppe, eine (C₁-C₄)-Alkylthioethergruppe, eine Mercaptogruppe, eine Nitrogruppe, eine Aminogruppe, eine (C₁-C₄)-Alkylaminogruppe, eine Di(C₁-C₄)alkylaminogruppe, eine Trifluormethangruppe, eine -C(O)H-Gruppe, eine -C(O)CH₃-Gruppe, eine -C(O)CF₃-Gruppe, eine -Si(CH₃)₃-Gnrppe, eine -C(O)-NH₂ Gruppe, eine (C₁-C₄)-Hydroxyalkylgruppe oder eine (C₂-C₄)-Dihydroxyalkylgruppe darstellen;
unter der Bedingung, dass (i) **R1** kein 2-Pyridylrest ist und (ii) **R1** kein 2-Hydroxy-4-amino-phenylrest ist und (iii) **R4** nicht gleich einer Aminogruppe ist, wenn gilt **R2=R3=R5=R6=H.**

## Claims

1. Agent for dyeing keratin fibres based on a developer substance-coupler substance combination, **characterized in that** it comprises at least one 3-aminophenol derivative of the formula (I) or physiologically compatible water-soluble salt thereof, 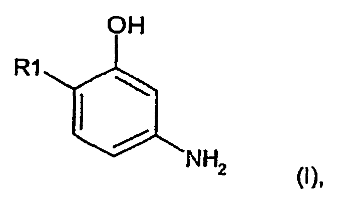 in which
**R1** is a radical of the formula (II) or (III), 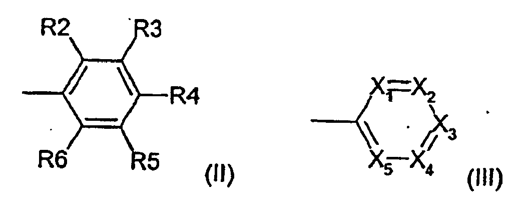 where **R2, R3, R4, R5** and **R6,** independently of one another, are hydrogen, a halogen atom, a cyano group, a hydroxy group, a C₁-C₄-alkoxy group, a phenoxy group, a C₁-C₄-hydroxyalkoxy group, a C₁-C₆-alkyl group, a phenyl group, a C₁-C₄-alkyl thioether group, a mercapto group, a nitro group, an amino group, a C₁-C₄-alkylamino group, a hydroxy(C₂-C₄) alkylamino group, a di(C₁-C₄) alkylamino group, a di(hydroxy(C₂-C₄)alkyl)amino group, a (dihydroxy(C₃-C₄) alkyl) amino group, a (hydroxy(C₂₋C₄)alkyl) -C₁-C₄-alkylamino group, a trifluoromethane group, a -C(O)H group, a -C(O)CH₃ group, a -C(O)CF₃ group, an -Si(CH₃)₃ group, a (C₁-C₄)-hydroxyalkyl group, a (C₂-C₄)-dihydroxyalkyl group, a (C₁-C₄)-aminoalkyl group, or a (C₁-C₄)-cyanoalkyl group, or two adjacent radicals R2 to R6 form a -O-CH2-O bridge;
**X**_{**1**}**, X**_{**2**}**, X**_{**3**}**, X**_{**4**} and **X**_{**5**}**,** independently of one another, are nitrogen or a C-R7 group, C-R8 group, C-R9 group, C-R10 group or C-R11 group, with the proviso that at least one and at most three of the radicals **X1** to **X5** are nitrogen; and
**R7, R8, R9, R10** and **R11**, independently of one another, are hydrogen, a halogen atom, a cyano group, a C₁-C₆-alkyl group, a (C₁-C₄)-alkyl thioether group, a mercapto group, a nitro group, an amino group, a (C₁-C₄) -alkylamino group, a di(C₁-C₄)alkylamino group, a trifluoromethane group, a -C(O)H group, a -C(O)CH₃ group, a -C(O)CF₃ group, an -Si(CH₃)₃ group, a -C(O)-NH₂ group, a (C₁-C₄)-hydroxyalkyl group or a (C₂-C₄)-dihydroxyalkyl group.

2. Agent according to Claim 1, **characterized in that** for the compounds of the formula (I): (i) **R1** is a radical of the formula (II) where **R2** and **R6** are hydrogen or (ii) **R1** is a radical of the formula (III) where **X1** and **X5** are **C-R7** and **C-R11,** where **R7** and **R11** are hydrogen.

3. Agent according to Claim 1 or 2, **characterized in that** the compounds of the formula (I) are chosen from 4-amino-3'-methyl-[1,1'-biphenyl]-2-ol, 4-amino-4'-methyl-3'-nitro-[1,1'-biphenyl]-2-ol, 4-amino-[1,1'-biphenyl]-2,4'-diol, 5-amino-2-(3-pyridinyl)phenol and 5-amino-2-(5-pyrimidinyl)phenol, and physiologically compatible water-soluble salts thereof.

4. Agent according to one of Claims 1 to 3, **characterized in that** the 3-aminophenol derivative of the formula (I) is present in an amount of from 0.005 to 20 per cent by weight.

5. Agent according to one of Claims 1 to 4, **characterized in that** the developer substance is chosen from the group consisting of 1,4-diaminobenzene, 1,4-diamino-2-methylbenzene, 1,9-diamino-2,6-dimethylbenzene, 1,4-diamino-3,5-diethylbenzene, 1,4-diamino-2,5-dimethylbenzene, 1,4-diamino-2,3-dimethylbenzene, 2-chloro-1,4-diaminobenzene, 1,4-diamino-2-(thiophen-2-yl)benzene, 1,4-diamino-2-(thiophen-3-yl)benzene, 1,4-diamino-2-(pyridin-3-yl)benzene, 2,5-diaminobiphenyl, 1,4-diamino-2-methoxymethylbenzene, 1,4-diamino-2-aminomethylbenzene, 1,4-diamino-2-hydroxymethylbenzene, 1,4-diamino-2-(2-hydroxyethoxy)benzene, 2-(2-(acetylamino)ethoxy)-1,4-diaminobenzene, 4-phenylaminoaniline, 4-dimethylaminoaniline, 4-diethylaminoaniline, 4-dipropylaminoaniline, 4-[ethyl(2-hydroxyethyl)amino]-aniline, 4-[di(2-hydroxyethyl)amino]aniline, 4-[di(2-hydroxyethyl)amino]-2-methylaniline, 4-[(2-methoxyethyl)amino]aniline, 4-[(3-hydroxypropyl)amino]aniline, 4-[(2,3-dihydroxypropyl)amino]aniline, 1,4-diamino-2-(1-hydroxyethyl)benzene, 1,4-diamino-2-(2-hydroxyethyl)benzene, 1,4-diamino-2-(1-methylethyl)benzene, 1,3-bis[(4-aminophenyl)(2-hydroxyethyl)amino]-2-propanol, 1,4-bis[(4-aminophenyl)amino]butane, 1,8-bis(2,5-diaminophenoxy)-3,6-dioxaoctane, 4-aminophenol, 4-amino-3-methylphenol, 4-amino-3-(hydroxymethyl)phenol, 4-amino-3-fluorophenol, 4-methylaminophenol, 4-amino-2-(aminomethyl)phenol, 4-amino-2-(hydroxymethyl)phenol, 4-amino-2-fluorophenol, 4-amino-2-[(2-hydroxyethyl)amino]methylphenol, 4-amino-2-methylphenol, 4-amino-2-(methoxymethyl)phenol, 4-amino-2-(2-hydroxyethyl)phenol, 5-aminosalicylic acid, 2,5-diaminopyridine, 2,4,5,6-tetraaminopyrimidine, 2,5,6-triamino-4-(1H)-pyrimidone, 4-5-diamino-1-(2-hydroxyethyl)-1H-pyrazole, 4,5-diamino-1-(1-methylethyl)-1H-pyrazole, 4,5-diamino-1-[(4-methylphenyl)methyl]-1H-pyrazole, 1-[(4-chlorophenyl)methyl]-4,5-diamino-1H-pyrazole, 4,5-diamino-1-methyl-1H-pyrazole, 2-aminophenol, 2-amino-6-methylphenol, 2-amino-5-methylphenol and 1,2,4-trihydroxybenzene.

6. Agent according to one of Claims 1 to 5, **characterized in that** in addition to the compounds of the formula (I), it comprises at least one further known coupler substance which is chosen from the group consisting of N-(3-dimethylaminophenyl)urea, 2,6-diaminopyridine, 2-amino-4-[(2-hydroxyethyl)amino]-anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 1,3-diamino-4-(2,3-dihydroxypropoxy)benzene, 1,3-diamino-4-(3-hydroxypropoxy)benzene, 1,3-diamino-4-(2-methoxyethoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3-[di(2-hydroxyethyl)amino]aniline, 4-amino-2-di [(2-hydroxyethyl)amino]-1-ethoxybenzene, 5-methyl-2-(1-methylethyl)phenol, 3-[(2-hydroxyethyl)amino]-aniline, 3-[(2-aminoethyl)amino]aniline, 1,3-di(2,4-diaminophenoxy)propane, di(2,4-diaminophenoxy)methane, 1,3-diamino-2,4-dimethoxybenzene, 2,6-bis(2-hydroxyethyl)aminotoluene, 4-hydroxyindole, 3-dimethylamino-phenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 2-[(3-hydroxyphenyl)amino]acetamide, 5-[(2-hydroxyethyl)amino]-4-methoxy-2-methylphenol, 5-[(2-hydroxyethyl)amino]-2-methylphenol, 3-[(2-hydroxyethyl)amino] phenol, 3-[(2-methoxyethyl)amino]phenol, 5-amino-2-ethylphenol, 5-amino-2-methoxyphenol, 2-(4-amino-2-hydroxyphenoxy)ethanol, 5-[(3-hydroxypropyl)amino]-2-methylphenol, 3-[(2,3-dihydroxypropyl)amino]-2-methylphenol, 3-[(2-hydroxyethyl)amino]-2-methylphenol, 2-amino-3-hydroxpyridine, 2,6-dihydroxy-3,4-dimethylpyridine, 5-amino-4-chloro-2-methylphenol, 1-naphthol, 2-methyl-1-naphthol, 1,5-dihydroxynaphthalene, 1,7-dihydroxynaphthalene, 2,3-dihydroxynaphthalene, 2,7-dihydroxynaphthalene, 2-methyl-1-napthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxol, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole and 2,3-indolinedione.

7. Agent according to one of Claims 1 to 6, **characterized in that** the developer substances and coupler substances, based on the total amount of the colouring agent, are each present in a total amount of from 0.005 to 20 per cent by weight.

8. Agent according to one of Claims 1 to 7, **characterized in that** it additionally comprises at least one direct dye.

9. Agent according to one of Claims 1 to 8, **characterized in that** it has a pH of from 6.5 to 11.5.

10. Ready-to-use agent for the oxidative dyeing of keratin fibres, which comprises, in a medium suitable for the dyeing, at least one developer substance and at least one coupler substance, and at least one oxidizing agent, **characterized in that** it comprises, as coupler substance, at least one 3-aminophenol derivative of the formula (I) according to one of Claims 1 to 3.

11. Agent according to one of Claims 1 to 10, **characterized in that** it is a hair dye.

12. 3-Aminophenol derivative of the formula (I) or physiologically compatible water-soluble salt thereof, 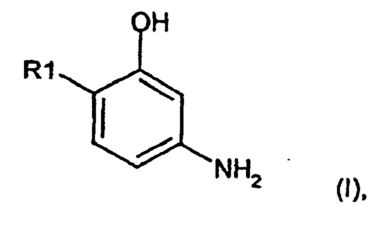 in which
**R1** is a radical of the formula (II) or (III), 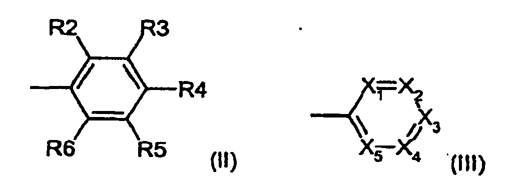 where **R2, R3, R4, R5** and **R6,** independently of one another, are hydrogen, a halogen atom, a cyano group, a hydroxy group, a C₁-C₄-alkoxy group, a phenoxy group, a C₁-C₄-hydroxyalkoxy group, a C₁-C₆-alkyl group, a phenyl group, a C₁-C₄-alkyl thioether group, a mercapto group, a nitro group, an amino group, a C₁-C₄-alkylamino group, a hydroxy(C₂-C₄)alkylamino group, a di(C₁-C₄)alkylamino group, a di(hydroxy (C₂-C₄)alkyl)amino group, a (dihydroxy(C₃-C₄)alkyl)amino group, a (hydroxy(C₂-C₄)alkyl)-C₁-C₄-alkylamino group, a trifluoromethane group, a -C(O)H group, a -C(O)CH₃ group, a -C(O)CF₃ group, an -Si(CH₃)₃ group, a (C₁-C₄)-hydroxyalkyl group, a (C₂-C₄)-dihydroxyalkyl group, a (C₁-C₄)-aminoalkyl group, or a (C₁-C₄)-cyanoalkyl group, or two adjacent radicals R2 to R6 form a -O-CH2-O bridge;
**X**_{**1**}**, X**_{**2**}**, X**_{**3**}**, X**_{**4**} and **X**_{**5**}**,** independently of one another, are nitrogen or a C-R7 group, C-R8 group, C-R9 group, C-R10 group or C-R11 group, with the proviso that at least one and at most three of the radicals **X1** to **X5** are nitrogen; and
**R7, R8, R9, R10** and **R11,** independently of one another, are hydrogen, a halogen atom, a cyano group, a C₁-C₆-alkyl group, a (C₁-C₄)-alkyl thioether group, a mercapto group, a nitro group, an amino group, a (C₁-C₄)-alkylamino group, a di(C₁-C₄)alkylamino group, a trifluoromethane group, a -C(O)H group, a -C(O)CH₃ group, a -C(O)CF₃ group, an -Si(CH₃)₃ group, a -C(O)-NH₂ group, a (C₁-C₉)-hydroxyalkyl group or a (C₂-C₄)-dihydroxyalkyl group;
with the proviso that (i) **R1** is not a 2-pyridyl radical and (ii) **R1** is not a 2-hydroxy-4-aminophenyl radical and (iii) **R4** is not an amino group if **R2=R3=R5=R6**=H.

## Revendications

1. Composition pour la teinture de fibres de kératine, à base d'une association développeur-coupleur, **caractérisée en ce qu'**elle contient au moins un dérivé de 3-aminophénol de formule (I) ou un sel hydrosoluble, physiologiquement acceptable, d'un tel dérivé, 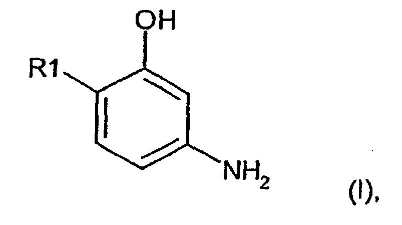 formule dans laquelle
**R1** représente un radical de formule (II) ou (III), 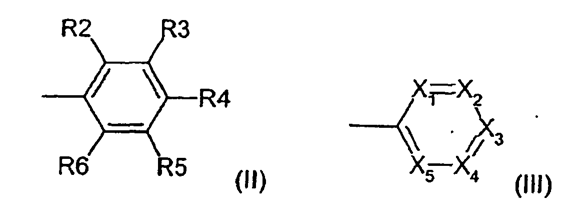 dans lesquelles **R2, R3, R4, R5** et **R6** représentent, indépendamment les uns des autres, un atome d'hydrogène, un atome d'halogène, un groupe cyano, un groupe hydroxy, un groupe alcoxy en C₁-C₄, un groupe phénoxy, un groupe hydroxyalcoxy en C₁-C₄, un groupe alkyle en C₁-C₆, un groupe phényle, un groupe alkyl(C₁-C₄)thioéther, un groupe mercapto, un groupe nitro, un groupe amino, un groupe alkyl(C₁-C₄)amino, un groupe hydroxyalkyl(C₂-C₄)amino, un groupe dialkyl(C₁-C₄)amino, un groupe di[hydroxyalkyl(C₂-C₄)]amino, un groupe [dihydroxyalkyl(C₃-C₄)]amino, un groupe [hydroxyalkyl(C₂-C₄)]-alkyl(C₁-C₄)amino, un groupe trifluorométhane, un groupe -C(O)H, un groupe -C(O)CH₃, un groupe -C(O)CF₃, un groupe -Si(CH₃)₃, un groupe hydroxyalkyle en C₁-C₄, un groupe dihydroxyalkyle en C₂-C₄, un groupe aminoalkyle(C₁-C₄) ou un groupe cyanoalkyle(C₁-C₄), ou deux radicaux R2 à R6 contigus forment un pont -O-CH₂-O- ;
**X**_{**1**}**, X**_{**2**}**, X**_{**3**}**, X**_{**4**} et **X**_{**5**} représentent, indépendamment les uns des autres un atome d'azote ou un groupe C-R7, un groupe C-R8, un groupe C-R9, un groupe C-R10 ou un groupe C-R11, étant entendu qu'au moins un et au maximum trois des radicaux **X1** à **X5** représente(nt) un atome d'azote ; et
**R7, R8, R9, R10** et **R11** représentent, indépendamment les uns des autres, un atome d'hydrogène, un atome d'halogène, un groupe cyano, un groupe alkyle en C₁-C₆, un groupe alkyl(C₁-C₄)thioéther, un groupe mercapto, un groupe nitro, un groupe amino, un groupe alkyl(C₁-C₄)amino, un groupe dialkyl(C₁-C₄)amino, un groupe trifluorométhane, un groupe -C(O)H, un groupe -C(O)CH₃, un groupe -C(O)CF₃, un groupe -Si(CH₃)₃, un groupe -C(O)-NH₂, un groupe hydroxyalkyle en C₁-C₄ ou un groupe dihydroxyalkyle en C₂-C₄.

2. Composition selon la revendication 1, **caractérisée en ce que** dans les composés de formule (I) les symboles ont les significations suivantes : (i) **R1** représente un radical de formule (II) où **R2** à **R6** représentent un atome d'hydrogène ou (ii) **R1** représente un radical de formule (III) où **X1** à **X5** représentent C-R7 et C-R11, R7 et R11 étant des atomes d'hydrogène.

3. Composition selon la revendication 1 ou 2, **caractérisée en ce que** les composés de formule (I) sont choisis parmi le 4-amino-3'-méthyl-[1,1'-biphényl]-2-ol, le 4-amino-4'-méthyl-3'-nitro[1,1'-biphényl]-2-ol, le 4-amino-[1,1'-biphényl]-2,4'-diol, le 5-amino-2-(3-pyridinyl)-phénol et le 5-amino-2-(5-pyrimidinyl)-phénol ainsi que leurs sels hydrosolubles physiologiquement acceptables.

4. Composition selon l'une quelconque des revendications 1 à 3, **caractérisée en ce que** le dérivé de 3-aminophénol de formule (I) est contenu en une quantité totale de 0,005 à 20 % en poids.

5. Composition selon l'une quelconque des revendications 1 à 4, **caractérisée en ce que** le développeur est choisi dans le groupe constitué par le 1,4-diaminobenzène, le 1,4-diamino-2-méthylbenzène, le 1,4-diamino-2,6-diméthylbenzène, le 1,4-diamino-3,5-diéthylbenzène, le 1,4-diamino-2,5-diméthylbenzène, le 1,4-diamino-2,3-diméthylbenzène, le 2-chloro-1,4-diaminobenzène, le 1,4-diamino-2-(thiophén-2-yl)benzène, le 1,4-diamino-2-(thiophén-3-yl)benzène, le 1,4-diamino-2-(pyridin-3-yl)benzène, le 2,5-diaminobiphényle, le 1,4-diamino-2-méthoxyméthylbenzène, le 1,4-diamino-2-aminométhylbenzène, le 1,4-diamino-2-hydroxyméthylbenzène, le 1,4-diamino-2-(2-hydroxyéthoxy)benzène, le 2-(2-(acétylamino)-éthoxy)-1,4-diaminobenzène, la 4-phénylaminoaniline, la 4-diméthylamino-aniline, la 4-diéthylamino-aniline, la 4-dipropylaminoaniline, la 4-[éthyl-(2-hydroxyéthyl)amino]aniline, la 4-[di(2-hydroxyéthyl)amino] aniline, la 4-[di(2-hydroxyéthyl)amino]-2-méthylaniline, la 4-[(2-méthoxyéthyl)amino]aniline, la 4-[(3-hydroxypropyl)amino]aniline, la 4-[(2,3-dihydroxypropyl] amino] aniline, le 1,4-diamino-2-(1-hydroxyéthyl)benzène, le 1,4-diamino-2-(2-hydroxyéthyl)benzène, le 1,4-diamino-2-(1-méthyléthyl)-benzène, le 1,3-bis[(4-aminophényl)(2-hydroxyéthyl)amino]-2-propanol, le 1,4-bis[(4-aminophényl)amino]butane, le 1,8-bis(2,5-diaminophénoxy)-3,6-dioxaoctane, le 4-aminophénol, le 4-amino-3-méthylphénol, le 4-amino-3-(hydroxyméthyl)phénol, le 4-amino-3-fluorophénol, le 4-méthylaminophénol, le 4-amino-2-(aminométhyl)-phénol, le 4-amino-2-(hydroxyméthyl)phénol, le 4-amino-2-fluorophénol, le 4-amino-2-[(2-hydroxyéthyl)amino]méthylphénol, le 4-amino-2-méthylphénol, le 4-amino-2-(méthoxyméthyl)phénol, le 4-amino-2-(2-hydroxyéthyl)phénol, l'acide 5-aminosalicylique, la 2,5-diaminopyridine, la 2,4,5,6-tétraaminopyrimidine, la 2,5,6-triamino-4(1H)-pyrimidone, le 4,5-diamino-1-(2-hydroxyéthyl)-1H-pyrazole, le 4,5-diamino-1-(1-méthyléthyl)-1H-pyrazole, le 4,5-diamino-1-[(4-méthylphényl)méthyl]-1H-pyrazole, le 1-[(4-chlorophényl)-méthyl]-4,5-diamino-1H-pyrazole, le 4,5-diamino-1-méthyl-1H-pyrazole, le 2-aminophénol, le 2-amino-6-méthylphénol, le 2-amino-5-méthylphénol et le 1,2,4-trihydroxybenzène.

6. Composition selon l'une quelconque des revendications 1 à 5, **caractérisée en ce qu'**elle contient, en plus des composés de formule (I), au moins un autre coupleur connu, qui est choisi dans le groupe constitué par la N-(3-diméthylaminophényl)urée, la 2,6-diaminopyridine, le 2-amino-4-[(2-hydroxyéthyl)amino]anisole, le 2,4-diamino-1-fluoro-5-méthylbenzène, le 2,4-diamino-1-méthoxy-5-méthylbenzène, le 2,4-diamino-1-éthoxy-5-méthylbenzène, le 2,4-diamino-1-(2-hydroxyéthoxy)-5-méthylbenzène, le 2,4-di[(2-hydroxyéthyl)amino]-1,5-diméthoxybenzène, la 2,3-diamino-6-méthoxypyridine, la 3-amino-6-méthoxy-2-(méthylamino)pyridine, la 2,6-diamino-3,5-diméthoxypyridine, la 3,5-diamino-2,6-diméthoxypyridine, le 1,3-diaminobenzole, le 2,4-diamino-1-(2-hydroxyéthoxy)benzène, le 1,3-diamino-4-(2,3-dihydroxypropoxy)benzène, le 1,3-diamino-4-(3-hydroxypropoxy)benzène, le 1,3-diamino-4-(2-méthoxyéthoxy)benzène, le 2,4-diamino-1,5-di(2-hydroxyéthoxy)benzène, le 1-(2-aminoéthoxy)-2,4-diaminobenzène, le 2-amino-1-(2-hydroxyéthoxy)-4-méthylaminobenzène, l'acide 2,4-diaminophénoxyacétique, la 3-[di(2-hydroxyéthyl)-amino]aniline, le 4-amino-2-di[(2-hydroxyéthyl)-amino]-1-éthoxybenzène, le 5-méthyl-2-(1-méthyléthyl)phénol, la 3-[(2-hydroxyéthyl)amino] aniline, la 3-[(2-aminoéthyl)amino]aniline, le 1,3-di(2,4-diaminophénoxy)propane, le di(2,4-diaminophénoxy)-méthane, le 1,3-diamino-2,4-diméthoxybenzène, le 2,6-bis(2-hydroxyéthyl)aminotoluène, le 4-hydroxyindole, le 3-diméthylaminophénol, le 3-diéthylaminophénol, le 5-amino-2-méthylphénol, le 5-amino-4-fluoro-2-méthylphénol, le 5-amino-4-méthoxy-2-méthylphénol, le 5-amino-4-éthoxy-2-méthylphénol, le 3-amino-2,4-dichlorophénol, le 5-amino-2,4-dichlorophénol, le 3-amino-2-méthylphénol, le 3-amino-2-chloro-6-méthylphénol, le 3-aminophénol, le 2-[(3-hydroxyphényl)amino]-acétamide, le 5-[(2-hydroxyéthyl)amino]-4-méthoxy-2-méthylphénol, le 5-[(2-hydroxyéthyl)amino]-2-méthylphénol, le 3-[(2-hydroxyéthyl)amino]phénol, le 3-[(2-méthoxyéthyl)amino]phénol, le 5-amino-2-éthylphénol, le 5-amino-2-méthoxyphénol,le 2-(4-amino-2-hydroxyphénoxy)éthanol, le 5-[(3-hydroxypropyl)amino]-2-méthylphénol, le 3-[(2,3-dihydroxypropyl)amino]-2-méthylphénol, le 3-[(2-hydroxyéthyl)amino]-2-méthylphénol, la 2-amino-3-hydroxypyridine, la 2,6-dihydroxy-3,4-diméthylpyridine, le 5-amino-4-chloro-2-méthylphénol, le 1-naphtol, le 2-méthyl-1-naptol, le 1,5-dihydroxynaphtalène, le 1,7-dihydroxynaphtalène, le 2,3-dihydroxynaphtalène, le 2,7-dihydroxynaphtalène, l'acétate de 2-méthyl-1-naphtol, le 1,3-dihydroxybenzène, le 1-chloro-2,4-dihydroxybenzène, le 2-chloro-1,3-dihydroxybenzène, le 1,2-dichloro-3,5-dihydroxy-4-méthylbenzène, le 1,5-dichloro-2,4-dihydroxybenzène, le 1,3-dihydroxy-2-méthylbenzène, le 3,4-méthylènedioxyphénol, la 3,4-méthylènedioxyaniline, le 5-[(2-hydroxyéthyl)-amino]-1,3-benzodioxole, le 6-bromo-1-hydroxy-3,4-méthylènedioxybenzène, l'acide 3,4-diaminobenzoique, la 3,4-dihydro-6-hydroxy-1,4(2H)-benzoxazine, la 6-amino-3,4-dihydro-1,4(2H)-benzoxazine, la 3-méthyl-1-phényl-5-pyrazolone, le 5,6-dihydroxyindole, la 5,6-dihydroxyindoline, le 5-hydroxyindole, le 6-hydroxyindole, le 7-hydroxyindole et la 2,3-indolinedione.

7. Composition selon l'une quelconque des revendications 1 à 6, **caractérisée en ce que** les développeurs et coupleurs sont contenus chacun, par rapport à la quantité totale de la composition de teinture, en une quantité totale de 0,005 à 20 % en poids.

8. Composition selon l'une quelconque des revendications 1 à 7, **caractérisée en ce qu'**en outre elle contient au moins un colorant direct.

9. Composition selon l'une quelconque des revendication 1 à 8, **caractérisée en ce qu'**elle présente un pH de 6,5 à 11,5.

10. Composition prête à l'emploi, pour la teinture par oxydation de fibres de kératine, qui contient dans un milieu approprié à la teinture, au moins un développeur et au moins un coupleur ainsi qu'au moins un oxydant, **caractérisée en ce qu'**elle contient au moins un dérivé de 3-aminophénol de formule (I) selon l'une quelconque des revendications 1 à 3.

11. Composition selon l'une quelconque des revendications 1 à 10, **caractérisée en ce qu'**il s'agit d'une composition de teinture pour cheveux.

12. Dérivé de 3-aminophénol de formule (I) ou sel hydrosoluble, physiologiquement acceptable, d'un tel dérivé, 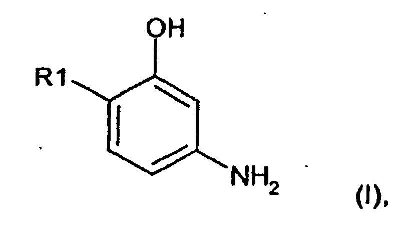 formule dans laquelle
**R1** représente un radical de formule (II) ou (III), 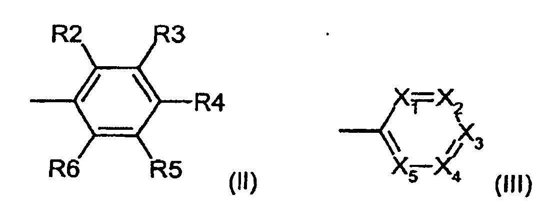 dans lesquelles **R2, R3, R4, R5** et **R6** représentent, indépendamment les uns des autres, un atome d'hydrogène, un atome d'halogène, un groupe cyano, un groupe hydroxy, un groupe alcoxy en C₁-C₄, un groupe phénoxy, un groupe hydroxyalcoxy en C₁-C₄, un groupe alkyle en C₁-C₆, un groupe phényle, un groupe alkyl(C₁-C₄)thioéther, un groupe mercapto, un groupe nitro, un groupe amino, un groupe alkyl(C₁-C₄)amino, un groupe hydroxyalkyl(C₂-C₄)amino, un groupe dialkyl(C₁-C₄)amino, un groupe di [hydroxyalkyl(C₂-C₄)]amino, un groupe [dihydroxyalkyl(C₃-C₄)]amino, un groupe [hydroxyalkyl(C₂-C₄)]alkyl(C₁-C₄)amino, un groupe trifluorométhane, un groupe -C(O)H, un groupe -C(O)CH₃, un groupe -C(O)CF₃, un groupe -Si (CH₃)₃, un groupe hydroxyalkyle en C₁-C₄, un groupe dihydroxyalkyle en C₂-C₄, un groupe aminoalkyle(C₁-C₄) ou un groupe cyanoalkyle (C₁-C₄), ou deux radicaux R2 à R6 contigus forment un pont -O-CH₂-O- ;
**X**_{**1**}**, X**_{**2**}**, X**_{**3**}**, X**_{**4**} et **X**_{**5**} représentent, indépendamment les uns des autres un atome d'azote ou un groupe C-R7, un groupe C-R8, un groupe C-R9, un groupe C-R10 ou un groupe C-R11, étant entendu qu'au moins un et au maximum trois des radicaux **X1** à **X5** représente(nt) un atome d'azote ; et **R7, R8, R9, R10** et **R11** représentent, indépendamment les uns des autres, un atome d'hydrogène, un atome d'halogène, un groupe cyano, un groupe alkyle en C₁-C₆, un groupe alkyl(C₁-C₄)thioéther, un groupe mercapto, un groupe nitro, un groupe amino, un groupe alkyl(C₁-C₄)amino, un groupe dialkyl (C₁-C₄)amino, un groupe trifluorométhane, un groupe -C(O)H, un groupe -C(O)CH₃, un groupe -C(O)CF₃, un groupe -Si(CH₃)₃, un groupe -C(O)-NH₂, un groupe hydroxyalkyle en C₁-C₄ ou un groupe dihydroxyalkyle en C₂-C₄ ;
étant entendu que (i) **R1** n'est pas un radical 2-pyridyle et (ii) **R1** n'est pas un radical 2-hydroxy-4-amino-phényle et (iii) **R4** n'est pas un groupe amino, lorsque **R2=R3=R5=R6=H**.
